# EUROPEAN PATENT APPLICATION

(11) **EP 4 206 320 A1**
(43) Date of publication of application: **05.07.2023**
(21) Application number: 21858393.8
(22) Date of filing: 20.08.2021
(51) Int. Cl.: C12N 5/074, C12M 3/00, C12N 5/079, C12P 7/44, C12P 13/08, C12P 13/24, C12P 17/10, C12P 19/32

(54) **METHOD OF CULTURING HUMAN INDUCED PLURIPOTENT STEM CELLS, CULTURE OF HUMAN INDUCED PLURIPOTENT STEM CELLS, AND METHOD OF PRODUCING CEREBRAL ORGANOIDS**

(30) Priority: 21.08.2020 JP 2020140133
(71) Applicant: JSR Corporation, Tokyo 105-8640 (JP); Keio University, Tokyo, 108-8345 (JP)
(72) Inventor: HIRAMINE, Hayato, Tokyo 105-8640 (JP); SHIOZAWA, Seiji, Tokyo 105-8640 (JP); OKANO, Hideyuki, Tokyo 160-8582 (JP); ISHIKAWA, Mitsuru, Tokyo 160-8582 (JP)
(74) Representative: TBK
(86) International application number: PCT/JP2021/030516
(87) International publication number: WO 2022/039250

(57) **Abstract**

A method of culturing human induced pluripotent stem cells includes a step of inoculating human induced pluripotent stem cells in a culture medium at an inoculation density of 1.0 × 10⁴ to 1.0 × 10⁶ cells/cm² in a culture vessel and subjecting the human induced pluripotent stem cells to two-dimensional culturing. A method of producing cerebral organoids includes a step 1 of culturing a culture of the human induced pluripotent stem cells obtained by the method of culturing human induced pluripotent stem cells in a culture medium containing a BMP inhibitor and a transforming growth factor β (TGFβ) inhibitor to form cell aggregates, a step 2 of culturing the cell aggregates in a culture medium containing a Wnt signal transduction pathway potentiator and an extracellular matrix, and a step 3 of subjecting the culturing obtained in the step 2 to spinner culturing.

## Description

### TECHNICAL FIELD

The present invention relates to a method of culturing human induced pluripotent stem cells, a culture of human induced pluripotent stem cells, and a method of producing cerebral organoids.

### BACKGROUND ART

Attempts have been made to utilize brains formed in vitro (cerebral organoids) for the development of therapeutic agents and diagnostic agents for brain diseases. For example, a method of culturing a cell mass of human induced pluripotent stem cells under high oxygen partial pressure conditions (Patent Document 1) or a method of culturing human induced pluripotent stem cells in an extracellular matrix (Patent Document 2) is known as a method of obtaining cerebral organoids.

### PRIOR ART LITERATURE

### Patent Document

Patent Document 1: United States Patent Application, Publication No. 2016/0289635
Patent Document 2: United States Patent No. 10407664

### DISCLOSURE OF INVENTION

### Problems to be Solved by the Invention

In the process of brain development, neural stem cells derived from the ectoderm immediately after closure of the neural tube take a morphology, in which neuroepithelial cells are budded layeredly, and then produce nerve cells by asymmetric division. That is, the formation of cell aggregates having neuroepithelial cells that have budded layeredly is necessary for the formation of cerebral organoids.

By the way, human induced pluripotent stem cells differ greatly in properties depending on the kind of cell line and number of passages. Therefore, in order to stably obtain cerebral organoids from human induced pluripotent stem cells, it is conceived to be preferable to keep the properties of the human induced pluripotent stem cells constant and ensure a state suitable for the formation of cell aggregates having neuroepithelial cells that have budded layeredly, in the production of cerebral organoids.

The present invention has been made in consideration of the above circumstances, and an object of the present invention is to provide a method of culturing human induced pluripotent stem cells suitable for the formation of cell aggregates having neuroepithelial cells that have budded layeredly, in the production of cerebral organoids, a culture of human induced pluripotent stem cells, and a method of producing cerebral organoids using the culture of human induced pluripotent stem cells.

### Means for Solving the Problems

That is, the present invention includes the following aspects.
(1) A method of culturing human induced pluripotent stem cells, including a step of inoculating human induced pluripotent stem cells in a culture medium at an inoculation density of 1.0 × 10⁴ to 1.0 × 10⁶ cells/cm² in a culture vessel and subjecting the human induced pluripotent stem cells to two-dimensional culturing.
(2) The method of culturing human induced pluripotent stem cells according to (1), in which the step of carrying out the two-dimensional culturing includes a step of carrying out culturing in a culture medium that contains a Rho kinase inhibitor.
(3) The method of culturing human induced pluripotent stem cells according to (1) or (2), in which the step of carrying out the two-dimensional culturing includes a step of carrying out culturing in a culture medium that does not contain a Rho kinase inhibitor.
(4) The method of culturing human induced pluripotent stem cells according to any one of (1) to (3), in which a confluency after the step of carrying out the two-dimensional culturing is 70% to 100% by area.
(5) The method of culturing human induced pluripotent stem cells according to any one of (1) to (4), in which the culture vessel is a culture vessel subjected to a surface treatment that improves cell adhesiveness.
(6) A method of producing cerebral organoids, including:
a step 1 of culturing a culture of the human induced pluripotent stem cells obtained by the method of culturing human induced pluripotent stem cells according to any one of (1) to (5) in a culture medium containing a BMP inhibitor and a transforming growth factor β (TGFβ) inhibitor to form cell aggregates;
a step 2 of culturing the cell aggregates in a culture medium containing a Wnt signal transduction pathway potentiator and an extracellular matrix; and
a step 3 of subjecting the culturing obtained in the step 2 to spinner culturing.

(7) The method of producing cerebral organoids according to (6), in which the spinner culturing in the step 3 is spinner culturing in a culture medium that does not contain the extracellular matrix.
(8) A culture of human induced pluripotent stem cells, which produces three or more metabolites selected from the group consisting of metabolites shown in Table 1, where a production amount of each of the metabolites is within a range shown in Table 1.

**[Table 1]**

| Metabolite | Production amount (pmol/10⁶ cells) |
|---|---|
| Glyceraldehyde 3-phosphate | 100 to 200 |
| Phosphocreatine | 150 to 400 |
| thymidine monophosphate (dTMP) | 7 to 20 |
| cytidine monophosphate (CMP) | 25 to 80 |
| uridine monophosphate (UMP) | 50 to 200 |
| Fructose 1,6-diphosphate | 800 to 2,000 |
| Adenosine monophosphate (AMP) | 150 to 500 |
| Inosine monophosphate (IMP) | 400 to 800 |
| guanosine triphosphate (GMP) | 30 to 100 |
| Uridine diphosphate (UDP) | 80 to 250 |
| Adenosine diphosphate (ADP) | 350 to 900 |
| Adenylosuccinic acid | 1 to 30 |
| Hydroxyproline | 8 to 20 |
| Creatine | 100 to 400 |
| 2-Aminoadipic acid | 100 to 350 |
| N⁶-Acetyllysine | 8 to 20 |
| N⁶,N⁶,N⁶-Trimethyllysine | 20 to 45 |
| Kynurenine | 20 to 45 |

(9) The culture of human induced pluripotent stem cells according to (8), in which the metabolites include adenylosuccinic acid.
(10) The culture of human induced pluripotent stem cells according to (8) or (9), in which the metabolites include inosine monophosphate.
(11) The culture of human induced pluripotent stem cells according to any one of (8) to (10), wherein the metabolites include adenosine monophosphate.

### Effects of the Invention

According to the method of culturing human induced pluripotent stem cells of the above aspect, it is possible to provide a method of culturing human induced pluripotent stem cells that form human induced pluripotent stem cells suitable for forming neuroepithelial cells, in the production of cerebral organoids.

### BRIEF DESCRIPTION OF THE DRAWINGS

FIG. 1 is a view representing bright field observation images and morphological evaluation of each cell aggregate in Experimental Example 2.
FIG. 2 is a view representing criteria for morphological evaluation of each cell aggregate in Experimental Example 2.
FIG. 3 is a view representing bright field observation images and morphological evaluation of each cell aggregate in Experimental Example 3.
FIG. 4 is graphs showing results of quantitative RT-PCR in Experimental Example 4.
FIG. 5 is a view showing immunostaining images of a human iPS culture of a sample A1 (low inoculation density: 1.4 × 10³ cells/cm²) in Experimental Example 4.
FIG. 6 is a view showing immunostaining images of a human iPS culture of a sample A5 (high inoculation density: 34.3 × 10³ cells/cm²) in Experimental Example 4.
FIG. 7A is a Heat Map of metabolome analysis according to HCA in Experimental Example 5.
FIG. 7B is a Heat Map of metabolome analysis according to HCA in Experimental Example 5.
FIG. 7C is a Heat Map of metabolome analysis according to HCA in Experimental Example 5.
FIG. 7D is a Heat Map of metabolome analysis according to HCA in Experimental Example 5.
FIG. 7E is a Heat Map of metabolome analysis according to HCA in Experimental Example 5.
FIG. 7F is a Heat Map of metabolome analysis according to HCA in Experimental Example 5.
FIG. 7G is a Heat Map of metabolome analysis according to HCA in Experimental Example 5.

### EMBODIMENTS FOR CARRYING OUT THE INVENTION

Hereinafter, the present invention will be described in more detail with reference to embodiments, but the present invention is not limited to the following embodiments.

Unless otherwise specified, each component exemplified in the present specification, for example, only one kind of component that is contained in a culture medium or only one kind of component that is used in each step can be used, or two or more kinds thereof can be used in combination.

In the present specification, the notation indicating a numerical range such as "A to B" is synonymous with "A or more and B or less", and A and B shall be included in the numerical range.

In the present specification, the terms "medium including substance X" and "in the presence of substance X" mean a culture medium to which an exogenous substance X has been added, a culture medium including an exogenous substance X, or in the presence of an exogenous substance X. That is, when a cell or tissue present in the culture medium expresses, secretes, or produces the substance X endogenously, the endogenous substance X is distinguished from the exogenous substance X, and it is noted that a culture medium that does not include the exogenous substance X does not fall under the category of "medium including substance X" even when the culture medium includes the endogenous substance X.

### <Method of culturing human induced pluripotent stem cells>

A method of culturing human induced pluripotent stem cells (hiPSCs) according to the present embodiment includes a step of inoculating human induced pluripotent stem cells in a culture medium at an inoculation density of 1.0 × 10⁴ to 1.0 × 10⁶ cells/cm² in a culture container (hereinafter also referred to as "culture vessel") and subjecting the human induced pluripotent stem cells to two-dimensional culturing.

In the method of culturing human induced pluripotent stem cells according to the present embodiment, in a case where the inoculation density of human induced pluripotent stem cells is within the above range, it is possible to produce a culture of human induced pluripotent stem cells which is suitable for forming neuroepithelial cells, in the production of cerebral organoids as shown in Examples below.

According to the method of culturing human induced pluripotent stem cells according to the present embodiment, it is suggested that intercellular signal transduction is important in the two-dimensional culturing of human induced pluripotent stem cells.

In a case where the inoculation density of the human induced pluripotent stem cells is less than the above lower limit value, the distance between the human induced pluripotent stem cells is long, and thus it is presumed that the intercellular signal transduction cannot sufficiently carried out until the human induced pluripotent stem cells proliferate. In addition, in a case where the inoculation density of the human induced pluripotent stem cells is less than the above lower limit value, the distances between the human induced pluripotent stem cells each vary during the process of the proliferation of the human induced pluripotent stem cells, and as a result, it is presumed that localization occurs in the concentration of intercellular transmitters and the like, and uniform intercellular signal transduction cannot be carried out.

On the other hand, in a case where the inoculation density of human induced pluripotent stem cells exceeds the above upper limit value, the time taken until being confluent is shortened, and thus it is presumed that intercellular signal transduction cannot be sufficiently carried out.

That is, in a case where the inoculation density of human induced pluripotent stem cells is within the above range, it is presumed that intercellular signal transduction can be carried out sufficiently, the localization of the concentration of intercellular transmitters is prevented, and as a result, it is possible to produce a culture of human induced pluripotent stem cells which is suitable for forming neuroepithelial cells, in the production of cerebral organoids.

Human induced pluripotent stem cells also include a genetically modified human induced pluripotent stem cell. The genetically modified human induced pluripotent stem cell can be prepared by transfecting ZFNs, TALEN, or CRISPR, which is an artificial nuclease, into human induced pluripotent stem cells. The artificial nuclease can introduce an insertion or deletion mutation by introducing a double strand DNA break (DSB) into a gene of interest and carrying out non-homologous end joining (NHEJ), which is one of the DSB repair mechanisms.

The human induced pluripotent stem cells mean cells in which pluripotency is induced by reprogramming somatic cells by a known method or the like. Specific examples thereof include cells obtained by reprograming differentiated somatic cells such as fibroblasts, peripheral blood mononuclear cells, or lymphocytes, by the expression of any combination of a plurality of genes selected from a group of reprogramming genes of Oct3/4, Sox2, Klf4, Myc (c-Myc, N-Myc, L-Myc), Glis1, Nanog, Sall4, Lin28, Esrrb, and the like, to induce the pluripotency.

The number of passages of human induced pluripotent stem cells is generally 1 to 100 times, preferably 5 to 80 times, and more preferably 10 to 40 times. In a case of using human induced pluripotent stem cells with the number of passages within the above range, it is possible to produce a culture of human induced pluripotent stem cells which is more suitable for forming neuroepithelial cells, in the production of cerebral organoids.

Two-dimensional culturing is a culturing method in which cells are two-dimensionally cultured in a state of adhering to a culture surface of a culture vessel or an extracellular matrix formed on a culture surface by a surface treatment.

Since the two-dimensional culturing according to the present embodiment allows human induced pluripotent stem cells to proliferate, the two-dimensional culturing can also be called expansion culturing.

In the two-dimensional culturing, the inoculation density of human induced pluripotent stem cells in a culture medium is 1.0 × 10⁴ to 1.0 × 10⁶ cells/cm², and it is preferably 2.0 × 10⁴ to 9.0 × 10⁵ cells/cm² and more preferably 3.0 × 10⁴ to 8.0 × 10⁵ cells/cm². In a case where the inoculation density of pluripotent stem cells is within the above range, it is possible to produce a culture of human induced pluripotent stem cells which is suitable for forming neuroepithelial cells, in the production of cerebral organoids.

The inoculation density can be calculated by dividing the number of cells to be inoculated (unit: cells) by the area (cm²) of the culturing surface of the culture vessel.

The culture vessel is preferably a culture vessel of which the culturing surface has been subjected to a surface treatment for improving cell adhesiveness. Examples of the surface treatment for improving adhesiveness include a coating treatment with laminin such as laminin α5β1γ1, laminin α1β1γ1, or laminin 511E8, entactin, an extracellular matrix such as collagen, gelatin, vitronectin, polylysine, or polyornithine, and a positive charge treatment.

Examples of the form of the culture vessel include a flask, a tissue culture flask, a culture dish, a tissue culture dish, a multi-dish, a microplate, a micro-well plate, a multi-plate, a multi-well plate, a chamber slide, a petri dish, a tube, a tray, a culture bag, a microcarrier, a stack plate, and a spinner flask.

A culture medium that is used in two-dimensional culturing (hereinafter, also referred to as an "expansion culturing medium") is preferably a feeder-free medium. Examples of the feeder-free medium include known culture media such as an hES9 medium, an hES9a culture medium, and an hESF-FX medium, and commercially available products such as TeSR-E8 (product name, manufactured by STEMCELL Technologies) and StemFit (registered trade name).

In the expansion culturing medium, in order to maintain differentiation potency, improve proliferation ability, and suppress cell death, human induced pluripotent stem cells can be cultured, before culturing in the feeder-free medium, in a culture medium which is the feeder-free medium containing a Rho kinase inhibitor (a ROCK inhibitor). That is, the two-dimensional culturing can include a step of carrying out culturing in a culture medium that contains a ROCK inhibitor. In a case where a culture medium obtained by adding a ROCK inhibitor to a feeder-free medium is used, the culturing period may be at most 5 days, and then the culturing is carried out in a feeder-free medium containing no ROCK inhibitor, generally for 1 day or more and preferably for 3 days or more. That is, the two-dimensional culturing can further include a step of carrying out culturing in a culture medium that does not contain a ROCK inhibitor.

The two-dimensional culturing preferably includes, in the following order; a step of carrying out culturing in a culture medium that contains a ROCK inhibitor and a step of carrying out culturing in a culture medium that does not contain a ROCK inhibitor.

Examples of the ROCK inhibitor include Y-27632 (CAS number: 129830-38-2), Fasudil/HA1077 (CAS number: 105628-07-7), H-1152 (CAS number: 871543-07-6), and Wf-536 (CAS number: 539857-64-2), as well as derivatives thereof.

The concentration of the ROCK inhibitor contained in the culture medium containing a ROCK inhibitor is generally such that the amount thereof provides a concentration of 0.1 µM or more and 100 µM or less, preferably such that the amount thereof provides a concentration of 1 µM or more and 80 µM or less, and more preferably such that the amount thereof provides a concentration of 5 µM or more and 50 µM or less.

The culture vessel during culturing is such that the temperature inside the container in which the culture vessel is placed is generally a temperature of 30°C or higher and 50°C or lower, preferably 32°C or higher and 48°C or lower, and more preferably 34°C or higher and 46°C or lower. The atmosphere of the culture vessel during culturing is such that the content proportion of the carbon dioxide inside the container in which the culture vessel is placed is generally 1% by volume or more and 15% by volume or less, preferably 2% by volume or more and 14% by volume or less, and more preferably 3% by volume or more and 13% by volume or less.

Human induced pluripotent stem cells can be dispersed before being subjected to two-dimensional culturing. The dispersion means separating cells into a cell population of 100 or less, preferably a cell population of 50 or less, and more preferably single cells, by a dispersion treatment such as an enzyme treatment or a physical treatment. Examples of the dispersion treatment include a mechanical dispersion treatment, a cell dispersion liquid treatment, and a treatment of adding a cell protecting agent. These treatments can be combined. Among these, a cell dispersion liquid treatment is preferable.

Examples of the cell dispersion liquid that is used in the cell dispersion liquid treatment include a solution containing any of enzymes such as trypsin, collagenase, hyaluronidase, elastase, pronase, DNase, and papain; and a chelating agent such as ethylenediaminetetraacetic acid. Examples of the commercially available cell dispersion liquid include TrypLE Select and TrypLE Express manufactured by Thermo Fisher Scientific, Inc. Examples of the mechanical dispersion treatment include a pipetting treatment and a scraping operation with a scraper. The dispersion treatment can be carried out by combining the cell dispersion liquid treatment and the mechanical dispersion treatment.

Before dispersing human induced pluripotent stem cells, a treatment with a cell protecting agent can be carried out to prevent cell death. Examples of the cell protecting agent include fibroblast growth factor (hereinafter, also referred to as "FGF"), heparin, a ROCK inhibitor, an insulin-like growth factor (hereinafter, also referred to as "1GF"), serum, and a serum substitute.

The confluency (the occupied area occupied by cells) after the two-dimensional culturing is preferably 70% to 100% by area, more preferably 75% to 100% by area, still more preferably 80% to 100% by area. In a case where the confluency is within the above range, it is possible to obtain a cell culture having more uniform properties.

It is noted that confluency referred to herein is the percentage of the proportion of the area occupied by the culture of human induced pluripotent stem cells after the two-dimensional culturing step with respect to the total area of the culturing surface of the culture vessel.

### <Culture of human induced pluripotent stem cells>

The culture of human induced pluripotent stem cells according to the present embodiment can be produced by the method of culturing human induced pluripotent stem cells according to the present embodiment.

The culture of human induced pluripotent stem cells according to the present embodiment produces three or more metabolites selected from the group consisting of metabolites shown in Table 2, where the production amount of each of the metabolites is within a range shown in Table 2, and the metabolites and the production amounts thereof can be checked by metabolome analysis. The metabolite candidates shown in Table 2 can be extracted by subjecting the results of the metabolome analysis of the culture of human induced pluripotent stem cells obtained at a low inoculation density and the culture of pluripotent stem cells according to the present embodiment to a hierarchical cluster analysis (abbreviated as "HCA").

Among the metabolites shown in Table 2, adenylosuccinic acid, inosine monophosphate (IMP), and adenosine monophosphate (AMP), which are related to purine metabolism, are particularly characteristical metabolites in the culture of human induced pluripotent stem cells according to the present embodiment, and the culture of human induced pluripotent stem cells is preferably such that at least one of these three has a production amount shown in Table 2.

**[Table 2]**

| Metabolite | Production amount (pmol/10⁶ cells) |
|---|---|
| Glyceraldehyde 3-phosphate | 100 to 200 |
| Phosphocreatine | 150 to 400 |
| thymidine monophosphate (dTMP) | 7 to 20 |
| cytidine monophosphate (CMP) | 25 to 80 |
| uridine monophosphate (UMP) | 50 to 200 |
| Fructose 1,6-diphosphate | 800 to 2,000 |
| Adenosine monophosphate (AMP) | 150 to 500 |
| Inosine monophosphate (IMP) | 400 to 800 |
| guanosine triphosphate (GMP) | 30 to 100 |
| Uridine diphosphate (UDP) | 80 to 250 |
| Adenosine diphosphate (ADP) | 350 to 900 |
| Adenylosuccinic acid | 1 to 30 |
| Hydroxyproline | 8 to 20 |
| Creatine | 100 to 400 |
| 2-Aminoadipic acid | 100 to 350 |
| N⁶-Acetyllysine | 8 to 20 |
| N⁶,N⁶,N⁶-Trimethyllysine | 20 to 45 |
| Kynurenine | 20 to 45 |

### <Method of producing cerebral organoids>

A method of producing a cerebral organoid according to the present embodiment has a step 1 of culturing a culture of human induced pluripotent stem cells obtained by the above-described method of culturing the culture of human induced pluripotent stem cells in a culture medium (hereinafter, also referred to as a "culture medium 1") containing a BMP inhibitor and a transforming growth factor β (TGFβ) inhibitor to form cell aggregates; a step 2 of culturing the cell aggregates in a culture medium (hereinafter, also referred to as a "culture medium 2") containing a Wnt signal transduction pathway potentiator and an extracellular matrix (hereinafter, also referred to as an "ECM"); and a step 3 of subjecting the culturing of the step 2 to spinner culturing.

According to the method of producing cerebral organoids according to the present embodiment, in the step 2, cell aggregates having neuroepithelial cells that have budded layeredly are formed in the culturing of the step 2. In addition, cerebral organoids in which telencephalon markers such as FOXG1 and SIX3 are sufficiently expressed can be formed.

The culturing in the step 1 and the step 2 is preferably suspension culturing.

The suspension culturing refers to carrying out culturing while maintaining a state in which cultured cells are suspended in a culture solution and a method of carrying out the culturing. The suspension culturing refers to culturing that is carried out under conditions in which cultured cells are not allowed to adhere to the culturing surface of the culture vessel.

The culture vessel that is used in the suspension culturing is preferably a culture vessel of which the culturing surface is cell non-adhesive. Examples of the culture vessel of which the culturing surface is cell non-adhesive include a flask, a tissue culture flask, a culture dish, a tissue culture dish, a multi-dish, a microplate, a micro-well plate, a multi-plate, a multi-well plate, a chamber slide, a petri dish, a tube, a tray, a culture bag, a microcarrier, a bead, a stack plate, a spinner flask, and a roller bottle and the like of which the surface has been subjected to a cell non-adhesive treatment with an MPC polymer or the like as well as those which have been processed into an unevenness shape.

### [Step 1]

In the step 1, a culture of human induced pluripotent stem cells is cultured in the culturing medium 1 to form cell aggregates. A cell aggregate exhibits a state in which two or more cells adhere to form an aggregate and is also referred to as a neurosphere.

As the culture vessel that is used for culturing in the culturing medium 1, a culture vessel having a narrow culture space can be used so that the cultured cells can aggregate to each other. Examples of the culture vessels having a narrow culture space include V-bottom plates of a 24-well plate (having an area of 1.88 cm² in terms of flat bottom), a 48-well plate (having an area of 1.0 cm² in terms of flat bottom), and a 96-well plate (having an area of 0.3 cm² in terms of flat bottom).

The culture medium 1 contains a BMP inhibitor and a TGFβ inhibitor. The culture medium 1 is generally prepared by adding a BMP inhibitor, a TGFβ inhibitor, and the like to a basal medium.

Examples of the basal medium include a BME culture medium, a BGJb medium, a CMRL 1066 medium, a Glasgow MEM (GMEM) culture medium, an Improved MEM Zinc Option medium, an IMDM medium, a Medium 199 medium, an Eagle MEM medium, an αMEM medium, a DMEM medium, an F-12 medium, a DMEMIF12 medium, an 1MDM/F12 medium, a Ham's medium, an RPM1 1640 medium, and a Fischer's medium; and a culture medium of a mixed culture medium thereof; as well as a culture medium obtained by reducing components related to neuronal differentiation from these culture medium. Among these, a culture medium obtained by reducing components related to neuronal differentiation is preferable.

Examples of the BMP inhibitor include chordin, nogin, follistatin, and dorsomorphin (6-[4-(2-piperidine-1-yl-ethoxy)phenyl]-3-pyridine-4-yl-pyrazolo[1,5-a]pyrimidine), DMH1 (4-[6-(4-isopropoxyphenyl)pyrazolo[1,5-a]pyrimidine-3-yl]quinoline, 4-[6-[4-(1-methylethoxy)phenyl]pyrazolo[1,5-a]pyrimidine-3-yl]-quinoline), and LDN193189 (4-(6-(4-(piperidine-1-yl)phenyl)pyrazolo[1,5-a]pyrimidine-3-yl)quinoline). Among these, dorsomorphin or LDN193189 is preferable.

The concentration of the BMP signaling pathway inhibitor contained in the culture medium 1 is preferably 0.5 µM or more and 10 µM or less, more preferably 0.75 µM or more and 5 µM or less, and still more preferably 1 µM or more and 3 µM or less.

The TGFβ inhibitor is a substance that inhibits the signal transduction pathway that is transduced by the Smad family, and examples of the TGF-β inhibitor include A83-01 (CAS number: 909910-43-6), SB-431542 (CAS number: 301836-41-9), SB-505124 (CAS number: 694433-59-5), SB-525334 (CAS number: 356559-20-1), LY364947 (CAS number: 396129-53-6), SD-208 (CAS number: 627536-09-8), and SJN2511 (CAS number: 446859-33-2). Among these, A83-01 or SB-431542 is preferable.

The concentration of the TGFβ inhibitor contained in the culture medium 1 is preferably 0.5 µM or more and 10 µM or less, more preferably 0.75 µM or more and 5 µM or less, and still more preferably 1 µM or more and 3 µM or less.

The culture medium 1 can further contain a neurobiological supplement, a culture medium supplement, serum, a serum substitute, and an antibacterial agent, as well as a serum-derived protein such as insulin or albumin.

Examples of the neurobiological supplement include a B27 supplement (product name, manufactured by Thermo Fisher Scientific, Inc.) containing biotin, cholesterol, linoleic acid, linolenic acid, progesterone, putrescine, retinol, retinyl acetate, sodium selenite, triiodothyronine (T3), DL-α-tocopherol (vitamin E), albumin, insulin, and transferrin; and an N2 supplement containing human transferrin, bovine insulin, progesterone, putrescine, and sodium selenite.

Examples of the culture medium supplement include a glutamic acid-containing supplement such as "GlutaMax series" (product name, manufactured by Thermo Fisher Scientific, Inc.) containing L-glutamic acid and a dipeptide obtained from L-glutamic acid, an amino acid solution such as "MEM Non-Essential Amino Acids Solution" (product name, manufactured by Thermo Fisher Scientific, Inc.), and 2-mercaptoethanol.

Examples of the antibacterial agent include a penicillin-based antibiotic, a cephem-based antibiotic, a macrolide antibiotic, a tetracycline-based antibiotic, a fosfomycin-based antibiotic, an aminoglycoside-based antibiotic, and a new quinolone antibiotic.

The culturing period is generally 1 day or more, preferably 3 days or more and 14 days or less, and more preferably 4 days or more and 12 days or less.

The culture vessel during culturing has generally a temperature of 30°C or higher and 50°C or lower, preferably 32°C or higher and 48°C or lower, and more preferably 34°C or higher and 46°C or lower. The atmosphere of the culture vessel during culturing is such that the content proportion of the carbon dioxide inside the container is generally 1% by volume or more and 15% by volume or less, preferably 2% by volume or more and 14% by volume or less, and more preferably 3% by volume or more and 13% by volume or less.

The culture of human induced pluripotent stem cells can be dispersed before being subjected to culturing in the culturing medium 1. The treatment method of dispersion is the same as the treatment method of dispersion described in the two-dimensional culturing step.

### [Step 2]

In the step 2, the cell aggregates obtained in the step 1 are cultured in the culturing medium 2 to form cell aggregates having neuroepithelial cells that have budded layeredly (hereinafter, also referred to as "cell aggregates of the step 2").

The step 2 can be carried out continuously by replacing only the culture medium without taking out the cell aggregates of the step 1 or can be carried out after taking out the cell aggregates of the step 1.

The culturing period is generally 1 day or more, preferably 3 days or more and 14 days or less, and more preferably 4 days or more and 12 days or less.

The culture vessel during culturing is such that the temperature inside the container in which the culture vessel is placed is generally a temperature of 30°C or higher and 50°C or lower, preferably 32°C or higher and 48°C or lower, and more preferably 34°C or higher and 46°C or lower. The atmosphere of the culture vessel during culturing is such that the content proportion of the carbon dioxide inside the container in which the culture vessel is placed is generally 1% by volume or more and 15% by volume or less, preferably 2% by volume or more and 14% by volume or less, and more preferably 3% by volume or more and 13% by volume or less.

The culture medium 2 is a culture medium containing a Wnt signal transduction pathway potentiator and ECM, which are related to the proliferation and maintenance of the undifferentiated state of stem cells. The culture medium 2 is generally prepared by adding a Wnt signal transduction pathway potentiator, ECM, and the like to a basal medium.

Examples of the basal medium include a BME medium, a BGJb medium, a CMRL1066 medium, GMEM (product name, manufactured by Thermo Fisher Scientific, Inc.), an Improved MEM Zinc Option medium (product name, manufactured by Thermo Fisher Scientific, Inc.), an IMDM medium, Medium 199 (product name, manufactured by Thermo Fisher Scientific, Inc.), an Eagle's MEM medium, an αMEM medium, a DMEM medium, a Ham's medium, a Ham's F-12 medium, and an RPMI1640 medium, as well as a mixture thereof.

Examples of the Wnt signal transduction pathway potentiator include a GSK-3β inhibitor, a Wnt protein, and a Wnt agonist. Among these, a GSK-3β inhibitor or a Wnt protein is preferable. The concentration of the Wnt signal transduction pathway potentiator contained in the culture medium 2 is generally 0.1 µM or more and 10 µM, and preferably 0.2 µM or more and 5 µM or less.

Examples of the GSK-3β inhibitor include CHIR99021 (CAS number: 252917-06-9), kenpaullone (CAS number: 142273-20-9), and 6-bromoindirubin-3'-oxime (BIO, CAS number: 667463-62-9). Among these, CHIR99021 is preferable.

The Wnt protein is preferably a Wnt protein derived from mammals. Examples of the mammalian Wnt protein include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. Among these, Wnt3a is preferred, and Wnt3a is more preferably a complex with afamin.

Examples of the method of carrying out culturing in a culture medium containing ECM include a method of embedding the cell aggregates of the step 1 in ECM and carrying out culturing thereof and a method of culturing the cell aggregates of the step 1 in a culture medium mixed with ECM. Among these, a method of culturing the cell aggregates of the step 1 in a culture medium mixed with ECM is preferable.

The culture medium mixed with ECM can be prepared by carrying out mixing so that the volume of ECM is generally 1% by volume or more, preferably 5% by volume or more and 100% by volume or less, and more preferably 10% by volume or more and 90% by volume or less with respect to the volume of components other than the ECM in the culture medium. Examples of the method of mixing ECM include a pipetting method on an ice bath. The mixing means that no ECM is visually observed in the culture medium.

Examples of the ECM include a component contained in the basement membrane and a glycoprotein present in the intercellular space. Examples of the component contained in the basement membrane include type IV collagen, laminin, heparan sulfate proteoglycan, and entactin. As the ECM, a commercially available product containing ECM can be used. Examples of the glycoprotein present in the intercellular space include collagen, laminin, entactin, fibronectin, and heparin sulfate. Examples of the commercially available product containing ECM include Matrigel (product name, manufactured by Corning Incorporated) and human type laminin (product name, manufactured by Sigma-Aldrich Co., LLC).

The culture medium 2 preferably further contains a TGFβ inhibitor. Examples of the TGFβ inhibitor include the same ones as those in the culture medium 1 described above. Among these, SB-431542 or SC-203294, which can selectively inhibit the kinase activity of ALK5, is preferable. The concentration of the TGF-β inhibitor contained in the culture medium 2 is generally 0.1 µM or more and 20 µM or less and preferably 0.1 µM or more and 10 µM or less.

The culture medium 2 can further contain a neurobiological supplement, a culture medium supplement, a serum-derived protein such as insulin or albumin, serum, and a serum substitute. The details of the neurobiological supplement and the culture medium supplement include the same ones as those shown as exemplary examples in the above-described culture medium 1.

### [Step 3]

In the step 3, the cell aggregates of the step 2 are matured through the spinner culturing to form glial cells, thereby forming cerebral organoids. Here, the culturing medium that is used in the spinner culturing is referred to as a culture medium 3.

In the step 3, the cell aggregates of the step 2 can be continuously cultured by replacing only the culturing medium from the culturing medium 2 to the culturing medium 3 without taking out the cell aggregates of the step 2 from the culture vessel, or the cell aggregates of the step 2 can be taken out from the culture vessel, transferred to another culture vessel, and then cultured in the culturing medium 3.

The culturing period is generally 1 day or more, preferably 2 days or more and 700 days or less, and more preferably 10 days or more and 365 days or less. The culture vessel during culturing is such that the temperature inside the container in which the culture vessel is placed is generally a temperature of 30°C or higher and 50°C or lower, preferably 32°C or higher and 48°C or lower, and more preferably 34°C or higher and 46°C or lower. The atmosphere of the culture vessel during culturing is such that the content proportion of the carbon dioxide inside the container in which the culture vessel is placed is generally 1% by volume or more and 15% by volume or less, preferably 2% by volume or more and 14% by volume or less, and more preferably 3% by volume or more and 13% by volume or less.

As the culture vessel that is used in the spinner culturing, a cell non-adhesive culture vessel is generally used.

The culture medium 3 generally contains a basal medium. In addition, the culture medium 3 is generally prepared by adding other components such as a neurobiological supplement, a culture medium supplement, a serum-derived protein such as insulin or albumin, serum, and a serum substitute, to a basal medium. Examples of the basal medium include those exemplified in the culture medium 2 described above.

It is preferable that the culture medium 3 contains substantially no ECM. "Containing substantially no ECM" means that no ECM is intendedly added to the culture medium 3, where ECM mixed as an unavoidable impurity is allowed.

The culture medium 3 can further contain at least one selected from the group consisting of a neurobiological supplement, a culture medium supplement, a serum-derived protein such as insulin or albumin, serum, and a serum substitute. The details of the neurobiological supplement and the culture medium supplement include the same ones as those described in the above-described culture medium 1.

The cerebral organoids produced by the method of producing cerebral organoids according to the present embodiment preferably contain telencephalon or telencephalon portion-like tissue. Here, the telencephalon portion-like tissue includes cerebral cortex, basal ganglia, hippocampus, choroid plexus, and the like. Here, whether or not a cerebral organoid contains the telencephalon or telencephalon portion-like tissue can be determined morphologically. Alternatively, the determination can be made by measuring the expression of a marker gene or marker protein characteristic of each tissue.

Examples of the telencephalon marker include FOXG1 and SIX3. In addition, examples of the cerebral cortex marker include CTIP2 which is a cerebral cortex layer V marker, and SATB2 which is a cerebral cortex layer 11/111 marker. In addition, examples of the basal ganglia marker include NKX2.1 and GSH2. In addition, examples of the hippocampus marker include KA1 and ZBTB2. In addition, examples of the choroid plexus marker include TTR and LMX1A.

It is preferable that in the cerebral organoid, the expression of OTX2, which is a forebrain and midbrain marker, is suppressed.

In a case where the culturing period in the step 3 is extended, more telencephalon or telencephalon portion-like tissue can be contained in the cerebral organoid.

### <Drug efficacy evaluation method>

A drug efficacy evaluation method includes a step of bringing the above-described cerebral organoid into contact with a test substance (hereinafter, also referred to as a "step A"), and a step of examining an effect of the test substance on the cerebral organoid (hereinafter, also referred to as a "step B").

In the step A, examples of the test substance include a natural compound library, a synthetic compound library, an existing drug library, and a metabolite library. The existing drugs include, for example, AZD2858 and a methylthioninium chloride hydrate. In addition, a new drug can be used as the test substance.

In the step B, the effect of the test substance on the cerebral organoid can be examined (evaluated) by Western blotting, ELISA, or immunostaining.

Further, before the step A, it is possible to provide a step of transplanting the cerebral organoid into the brain of a mammal (hereinafter, also referred to as a "step a"). In a case where cerebral organoids exhibit an Alzheimer's disease-like pathological condition, the drug efficacy of the test substance can be evaluated in an environment similar to that of a living body suffering from Alzheimer's disease in a case where the step a is provided.

### EXAMPLES

Hereinafter, the present embodiment will be described in more detail based on Examples. However, the present embodiment is not limited to these Examples.

### [Experimental Example 1]

### (Culturing of human iPS cells)

Human iPS cells (a PChiPS771 strain, Lot. A01QM28, manufactured by ReproCELL Inc.) were washed with phosphate buffered physiological saline (PBS) and then dispersed into single cells using TrypLE Select (manufactured by Thermo Fisher Scientific, Inc.). The dispersed human iPS cells were inoculated in a culture medium at the inoculation density shown in Table 3, and the culture vessel was placed in an incubator (37°C, 1 atm, CO₂ concentration: 5 v/v%) to carry out culturing.

A 60 mm dish (for cell culturing, manufactured by IWAKI & CO., LTD.) coated with a human recombinant laminin fragment (product name: "iMatrix-51 1 ", manufactured by Nippi. Inc.) containing only the active site of laminin-511 was used as the culture vessel, and a culture medium obtained by adding Y27632 (a ROCK inhibitor, concentration in culture medium: 10 µM) to a basal medium (StemFit AK02N medium, manufactured by Ajinomoto Co., Inc.) was used as the culturing medium.

One day after the start of culturing, the culturing medium was exchanged with a culture medium containing only a basal medium (StemFit AK02N medium, manufactured by Ajinomoto Co., Inc.) that did not contain Y27632. Thereafter, the culturing medium was exchanged with a culture medium containing only the basal medium every day and the culturing was carried out for 7 days to obtain each human iPS cell culture.

**[Table 3]**

| Sample | Inoculation density (× 10³ cells/cm²) |
|---|---|
| A1 | 1.4 |
| A2 | 4.3 |
| A3 | 8.6 |
| A4 | 17.1 |
| A5 | 34.3 |

### [Experimental Example 2]

### (Formation of cell aggregates having neuroepithelial cells that have budded layeredly)

The human iPS cell culture obtained in Experimental Example 1 was subjected to a cell dispersion liquid treatment by using TrypLE Select (product name, manufactured by Thermo Fisher Scientific, Inc.) and further dispersed into single cells by a pipetting operation. The dispersed human iPS cells were inoculated in a 96-well plate (product name: "PrimeSurface 96V bottom plate", manufactured by Sumitomo Bakelite Co., Ltd.) so that the cell density was 1 × 10⁴ cells/well in the culturing medium 1 of 100 µL/well, having the composition shown in Table 4, and were cultured in an incubator (37°C, 1 atm, CO₂ concentration: 5 v/v %) for 7 days (7 days after the start of culturing of the human iPS cells) to obtain aggregates.

The culturing medium 1 was removed, the culturing medium 2 having the composition shown in Table 5 was added to an amount of 150 µL/well, and the aggregates of the human iPS cells were further subjected to suspension culturing with stirring in an incubator (37°C, CO₂ concentration: 5 v/v%) for 7 days (14 days after the start of culturing of the human iPS cells) to form cell aggregates.

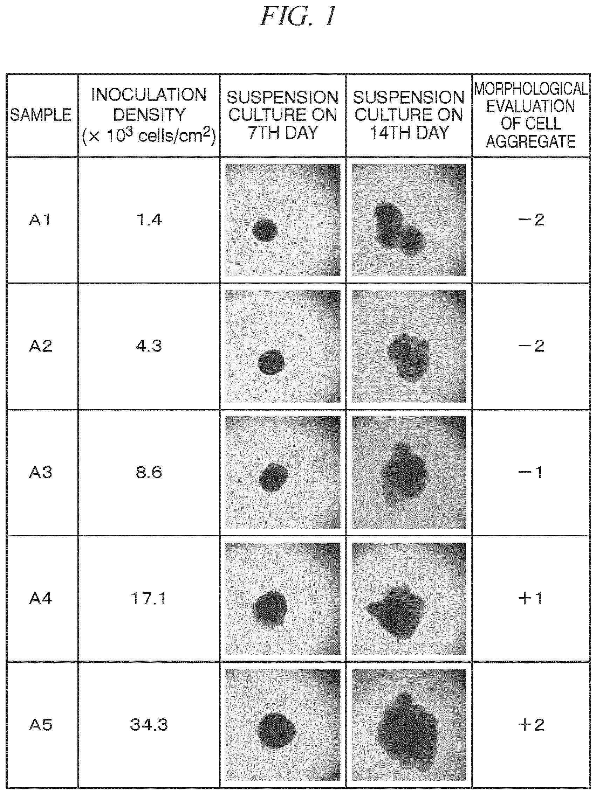

FIG. 1 shows bright field images obtained with an optical microscope and morphological evaluation of the cell aggregates on the 7th day and 14th day from the start of culturing of the human iPS cells.

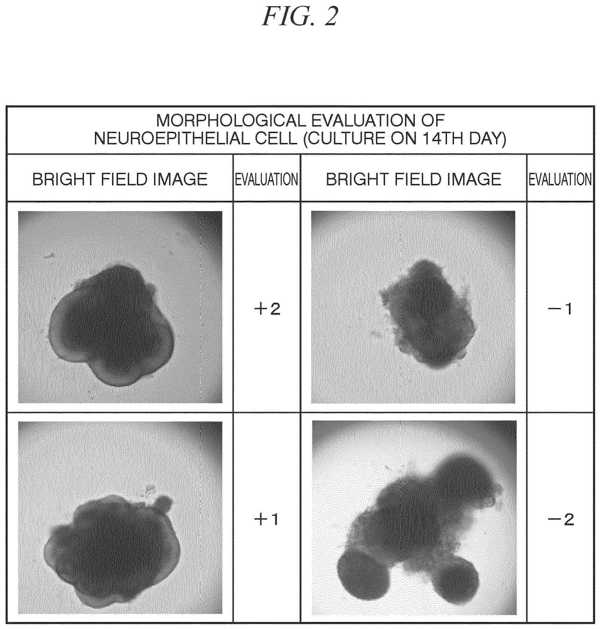

It is noted that the morphological evaluation of the cell aggregates was carried out based on the criteria shown in FIG. 2 using the bright field images obtained with an optical microscope on the 14th day after the start of culturing of the human iPS cells.

**[Table 4]**

| Component | | Content |
|---|---|---|
| Basal medium | StemFit AK02N (product name, manufactured by Ajinomoto Co., Inc.) | - |
| Culture medium supplement | MEM Non-Essential Amino Acids Solution (100X) (product name, manufactured by Thermo Fisher Scientific, Inc.) | Using amount: an amount that provides a concentration diluted by 200-fold with respect to the basal medium |
| | Penicillin-streptomycin mixed solution, sterilization test, mycoplasma, endotoxin tested (product name, manufactured by Nacalai Tesque Inc.) | Using amount: an amount that provides a concentration diluted by 100-fold with respect to the basal medium |
| | DMEM, low glucose, GlutaMAX^{™} Supplement, pyruvate (product name, manufactured by Thermo Fisher Scientific, Inc.) | Using amount: an amount that provides a concentration diluted by 100-fold with respect to the basal medium |
| | 2-mercaptoethanol (product name, manufactured by Thermo Fisher Scientific, Inc.) | Using amount: an amount that provides a concentration diluted by 1,000-fold with respect to the basal medium |
| BMP inhibitor | Dorsomorphin | Final concentration: 2 µM |
| TGF-β inhibitor | A83-01 | Final concentration: 2 µM |

**[Table 5]**

| Component | | Content |
|---|---|---|
| Basal medium | DMEM/F-12, HEPES (product name, manufactured by Thermo Fisher Scientific, Inc.) | - |
| Culture medium supplement | MEM Non-Essential Amino Acids Solution (100X) (product name, manufactured by Thermo Fisher Scientific, Inc.) | Using amount: an amount that provides a concentration diluted by 200-fold with respect to the basal medium |
| | Penicillin-streptomycin mixed solution, sterilization test, mycoplasma, endotoxin tested (product name, manufactured by Nacalai Tesque Inc.) | Using amount: an amount that provides a concentration diluted by 100-fold with respect to the basal medium |
| | DMEM, low glucose, GlutaMAX^{™} Supplement, pyruvate (product name, manufactured by Thermo Fisher Scientific, Inc.) | Using amount: an amount that provides a concentration diluted by 100-fold with respect to the basal medium |
| Wnt signal potentiator | Wnt-3a, Human, Recombinant (product name, manufactured by R&D Systems) | Final concentration: 4 ng/mL |
| | CHIR99021 | Final concentration: 1 µM |
| TGF-β inhibitor | SB-431542 | Final concentration: 1 µM |
| Extracellular matrix | Matrigel | Final concentration: 30% by volume |

**[Table 6]**

| Component | | Content |
|---|---|---|
| Basal medium | DMEMIF-12, HEPES (product name, manufactured by Thermo Fisher Scientific, Inc.) | - |
| Culture medium supplement | N-2 Supplement (100X) (product name, manufactured by Thermo Fisher Scientific, Inc.) | Using amount: an amount that provides a concentration diluted by 200-fold with respect to the basal medium |
| | B-27^{®} Serum-free Supplement (product name, manufactured by Thermo Fisher Scientific, Inc.) | Using amount: an amount that provides a concentration diluted by 100-fold with respect to the basal medium |
| | MEM Non-Essential Amino Acids Solution (100X) (product name, manufactured by Thermo Fisher Scientific, Inc.) | Using amount: an amount that provides a concentration diluted by 200-fold with respect to the basal medium |
| | 2-mercaptoethanol (product name, manufactured by Thermo Fisher Scientific, Inc.) | Using amount: an amount that provides a concentration diluted by 1,000-fold with respect to the basal medium |
| | Insulin Solution, Human, Recombinant (product name, manufactured by FUJIFILM Wako Pure Chemical Corporation) | Final concentration: 2.5 µg/mL |

From FIG. 1, it was shown that in the human iPS cell cultures with inoculation densities of 17.1 × 10³ cells/cm² and 34.3 × 10³ cells/cm² the morphological evaluation of the cell aggregate is +1 or more, which is favorable.

### [Experimental Example 3]

### (Influence of number of passages of human iPS cells)

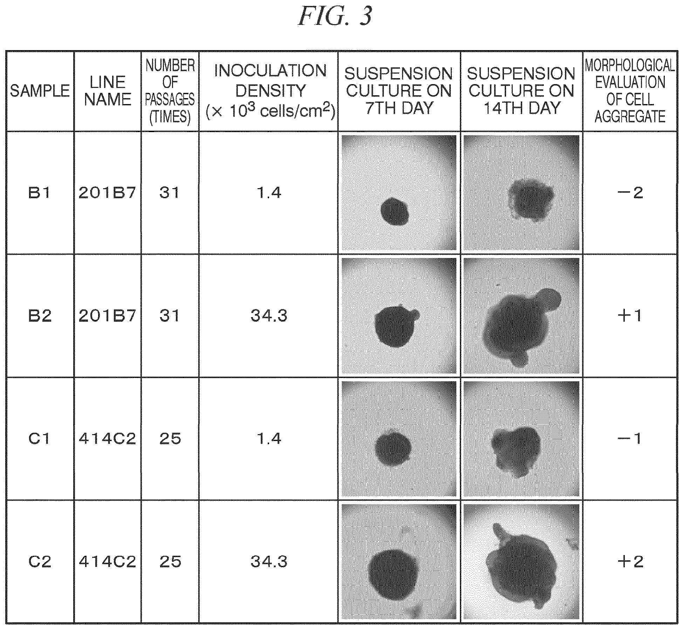

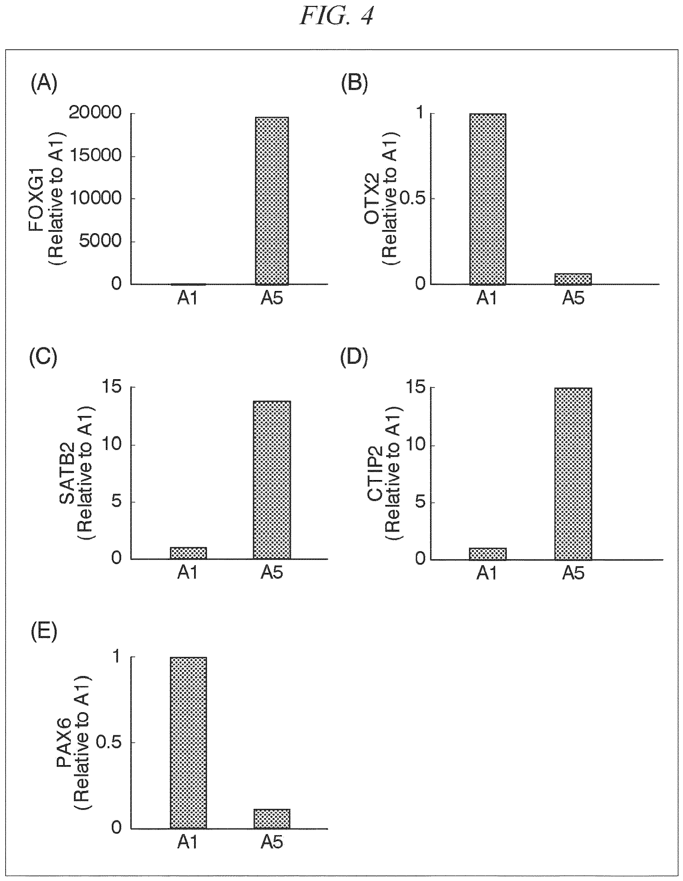

In Experimental Example 1, neuroepithelial cells were produced in the same manner as in Experimental Example 1 and Experimental Example 2, except that human iPS cells with the number of passages shown in FIG. 4 were used. FIG. 3 shows the bright field images obtained with the optical microscope and the morphological evaluation of the cell aggregates.

As shown in FIG. 3, in the human iPS cell culture with an inoculation density of 34.3 × 10³ cells/cm², the morphological evaluation of the cell aggregate was +1 or more in a case where human iPS cells with any number of passages were used, which was favorable.

### [Experimental Example 4]

### (Production of cerebral organoids and measurement of expressed genes thereof by RT-qPCR)

Cell aggregates were formed using human iPS cultures of a sample A1 (low inoculation density: 1.4 × 10³ cells/cm²) and a sample A5 (high inoculation density: 34.3 × 10³ cells/cm²), obtained in Experimental Example 1, according to the same operation as in Experimental Example 2.

Subsequently, the contents of the wells were transferred to 50 mL Falcon^{™} conical tubes (manufactured by Corning Incorporated) containing 10 mL of PBS. Subsequently, mixing with inversion was carried out 5 times, the culture medium 2 was removed by removing the supernatant, and the cell aggregates were collected. Thirty collected cell aggregates and 30 mL of the culturing medium 3 having the composition shown in Table 6 were added to a single-use bioreactor (ABLE Corporation) and subjected to suspension culturing with stirring to produce cerebral organoids. The culture medium was exchanged every 4 days.

The total RNA of the cerebral organoids was extracted using a commercially available kit (product name: "RNeasy PlusMini Kit", QIAGEN N.V.). In addition, the concentration of the total RNA was measured using NanoDrop (product name, Thermo Fisher Scientific, Inc.). Subsequently, the total RNA was reverse transcribed with RT Master Mix (product name, TOYOBO Co., Ltd.) and a Nuclease free water to synthesize cDNA.

Premix Ex Taq (product name, Takara Bio Inc.) and ROX Reference Dye II (product name, Takara Bio Inc.) were added to the above cDNA to prepare a reaction solution. Subsequently, using a real-time PCR system (product name "ViiA7", Thermo Fisher Scientific, Inc.), the RT-qPCR analysis of the reaction solution was carried out, and the expression levels of regional markers (telencephalon: FOXG1, retina: OTX2), nerve cell markers of the cerebral cortex (SATB2, CTIP2), and a neural stem cell marker (PAX6) were measured. The measurement results are shown in FIG. 4 ((A): FOXG1, (B): OTX2, (C): SATB2, (D): CTIP2, (E): PAX6). The expression level of cerebral organoids derived from the human iPS culture of the sample A1 (low inoculation density: 1.4 × 10³ cells/cm²) is set to 1, and the expression level of cerebral organoids derived from the human iPS culture of the sample A5 (high inoculation density: 34.3 × 10³ cells/cm²) is shown.

In a case of cerebral organoids derived from the human iPS culture of the sample A5 (high inoculation density: 34.3 × 10³ cells/cm²), the expression level of FOXG1 was high as compared with cerebral organoids derived from the human iPS culture of the sample A1 (low inoculation density: 1.4 × 10³ cells/cm²), whereas the expression level of OTX2 was low. This suggested that the differentiation to the retina has not proceeded as compared with cerebral organoids derived from the human iPS culture with the low inoculation density but that the differentiation to the telencephalon has proceeded. In addition, in cerebral organoids derived from the human iPS culture with the high inoculation density, the expression levels of SATB2 and CTIP2 were high, but the expression level of PAX6 was low as compared with cerebral organoids derived from the human iPS culture with the low inoculation density. This suggested that there are more cells differentiated into the cerebral cortex but there are fewer cells differentiated into the neural stem cells as compared with cerebral organoids derived from the human iPS culture with the low inoculation density.

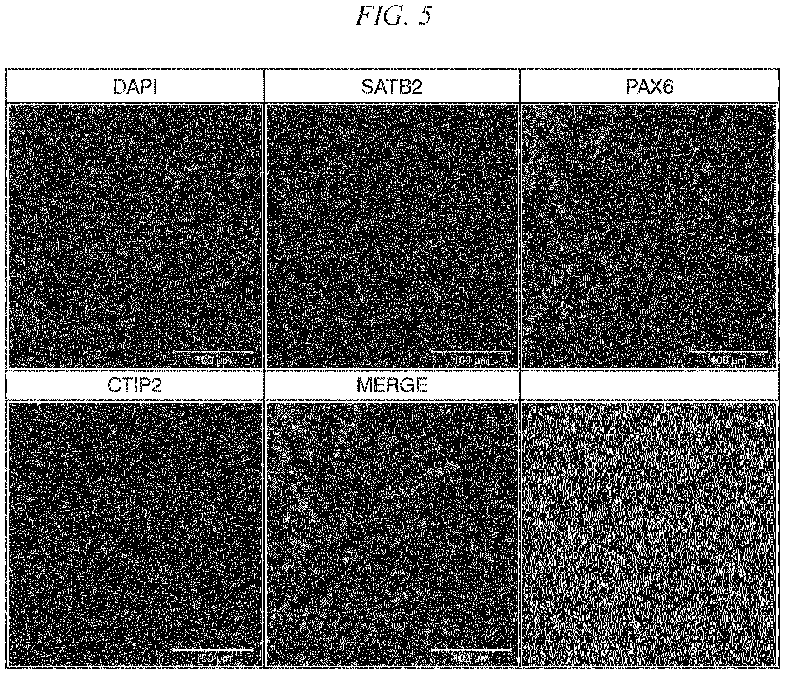

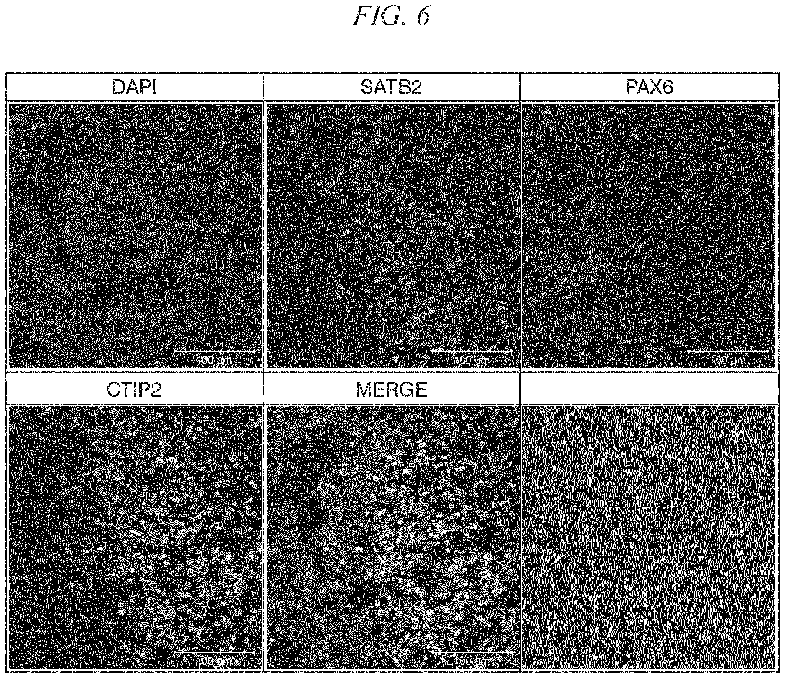

Further, by fluorescent immunostaining of sections of cerebral organoids derived from the human iPS cultures of the sample A1 (low inoculation density: 1.4 × 10³ cells/cm²) and the sample A5 (high inoculation density: 34.3 × 10³ cells/cm²), the expression of proteins of SATB2, CTIP2, and PAX6 was checked (see FIG. 5: fluorescent immunostaining images of cerebral organoids derived from the human iPS culture with the low inoculation density, and FIG. 6: fluorescent immunostaining images of cerebral organoids derived from the human iPS culture with the high inoculation density).

As shown in FIG. 5 and FIG. 6, regarding the protein expression level as well which was similar to the mRNA expression level, it was confirmed that, in cerebral organoids derived from the human iPS culture with the high inoculation density, the expression levels of SATB2 and CTIP2 are high, but the expression level of PAX6 is low as compared with cerebral organoids derived from the human iPS culture with the low inoculation density.

### [Experimental Example 5]

### (Measurement of metabolites of culture of pluripotent stem cells by metabolome analysis)

Each of human iPS cell cultures of sample names "30-3-13", "30-3-14", "30-3-15", "33-72-16", "33-72-17", and "33-72-18" was prepared in the same manner as in Experimental Example 1, except that the inoculation was carried out at the number of passages and inoculation density shown in Table 7 below.

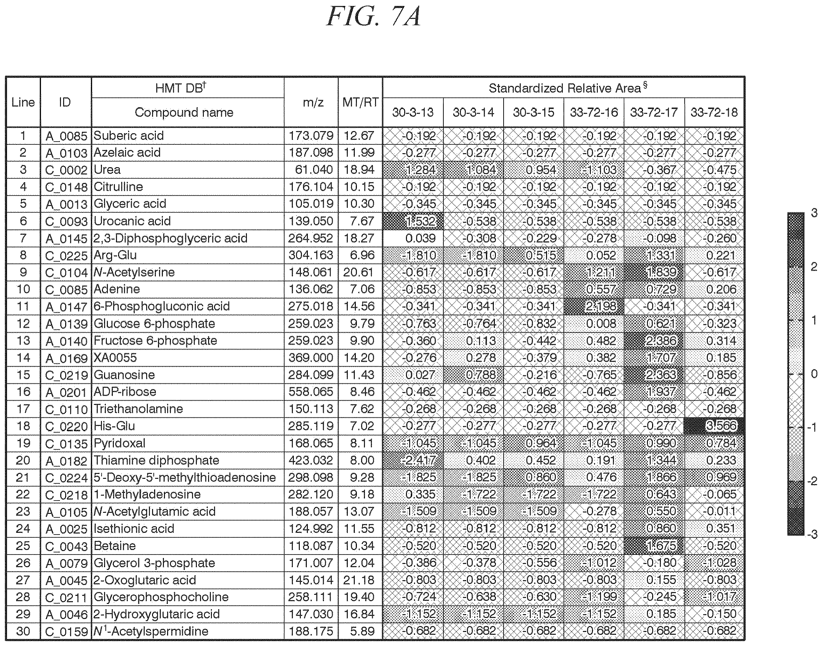

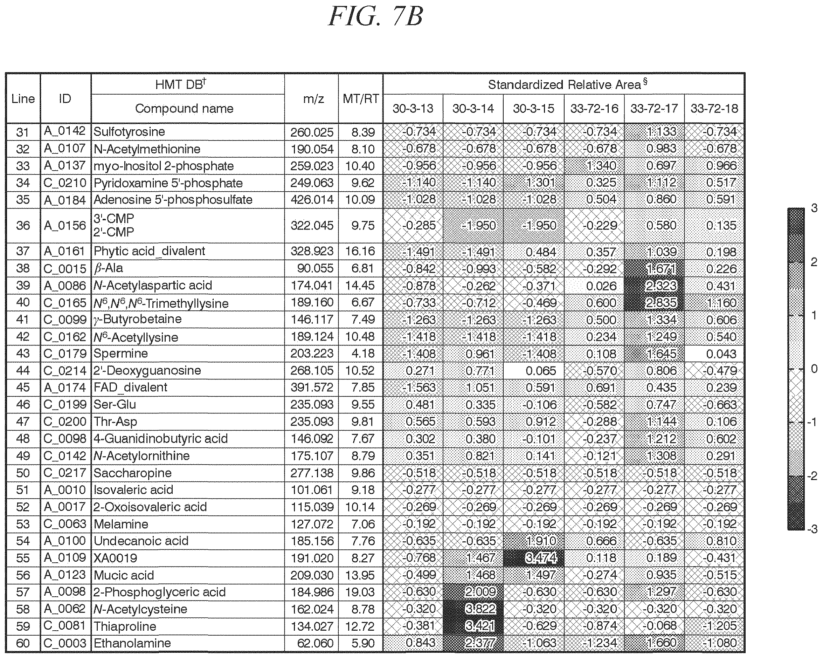

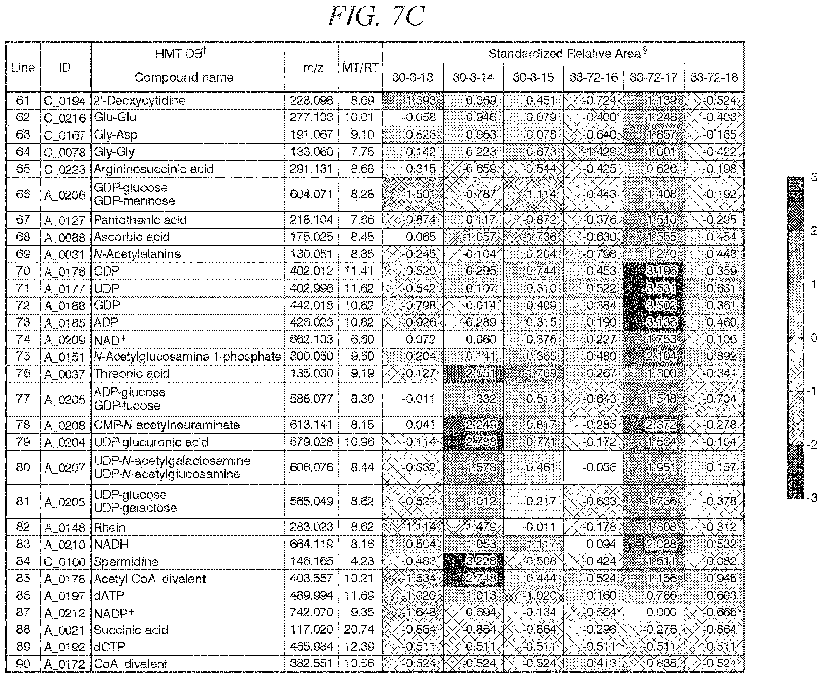

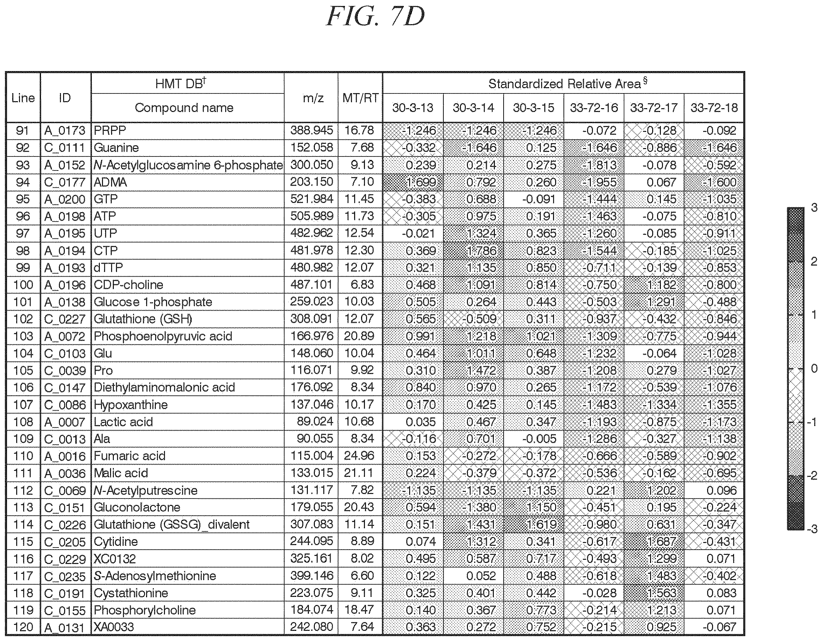

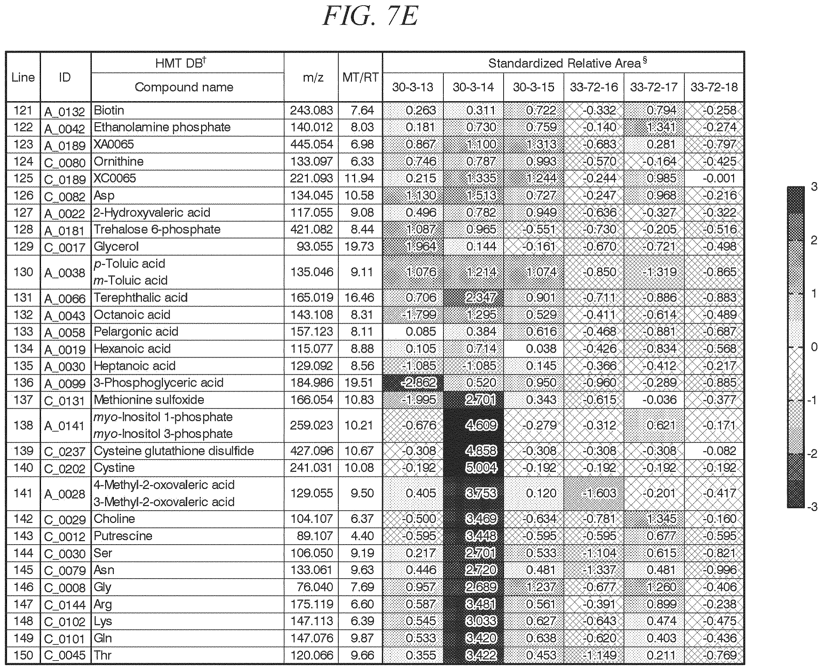

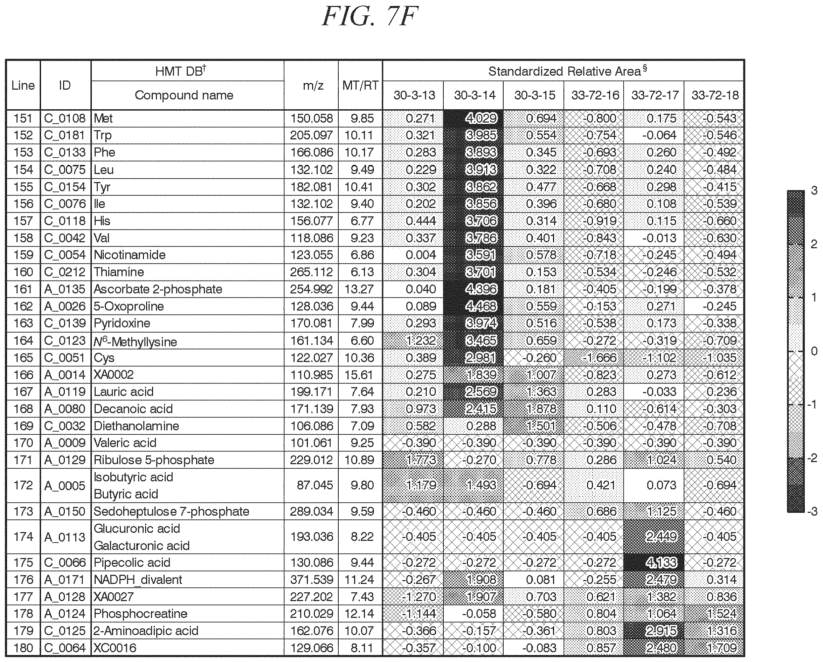

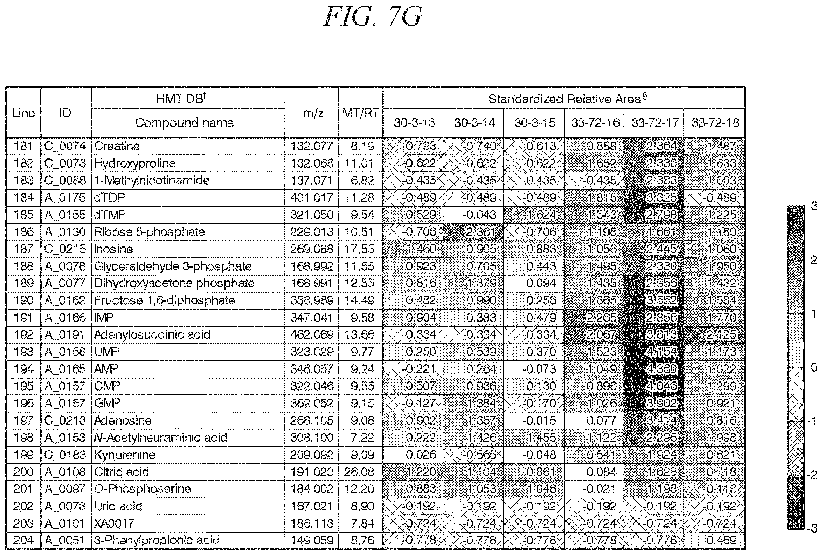

The metabolome analysis of each human iPS cell culture was carried out, and the measurement of the metabolite amounts (Table 8-1 to Table 8-14 and Table 9), and the creation of Heat Map according to HCA (FIG. 7A to FIG. 7G) were carried out. Table 8-1 to Table 8-14 show the total metabolite data of candidate compounds from the metabolome analysis of each of the human iPS cell cultures of sample names "30-3-13", "30-3-14", "30-3-15", "33-72-16", "33-72-17", and "33-72-18" in Experimental Example 5. Table 9 shows the results of carrying out the calculation with respect to metabolites which shows a variation in Table 8-1 to Table 8-14. In Table 8-1 to Table 8-14 and Table 9, ID consists of the first letter of the measurement mode and a serial number, where C indicates the cation mode, and A indicates the anion mode. "N.D." is an abbreviation for Not Detected and indicates that the corresponding metabolite is below the detection limit although it is an analysis target. "N.A." is an abbreviation for Not Available and indicates that the corresponding metabolite cannot be calculated due to lack of data although it is a calculation target. The ratio of detection average values between the two groups was calculated using the latter as the denominator. Welch's t-test p-values and ranges thereof were < 0.05 for *, < 0.01 for **, and < 0.001 for ***. In FIG. 7A to FIG. 7G, ID consists of the first letter of the measurement mode and a serial number, where C indicates the cation mode, and A indicates the anion mode. "Compound name" of "HMT DB" indicates a candidate compound obtained by matching the m/z and MT of the detected peak against the HMT database. "Standardized Relative Area" is a value obtained by standardizing the relative area of the detected peak, and eps (2-52) was substituted for the ND of the original data in order to calculate the distance.

**[Table 7]**

| Sample name | Inoculation density | Number of passages |
|---|---|---|
| 30-3-13 | 3 × 10⁴ | 30 |
| 30-3-14 | 3 × 10⁴ | 30 |
| 30-3-15 | 3 × 10⁴ | 30 |
| 33-72-16 | 72 × 10⁴ | 33 |
| 33-72-17 | 72 × 10⁴ | 33 |
| 33-72-18 | 72 × 10⁴ | 33 |

**[Table 8-1]**

| ID | Metabolite | Concentration (pmol/10⁸ cells) | | | | | |
|---|---|---|---|---|---|---|---|
| | | 30--3--13 | 30-3-14 | 30-3-15 | 33-72-16 | 33-72-17 | 33-72-18 |
| C_0007 | 1,3-Diaminopropane | N.D. | N.D. | N.D. | N.D. | N.D. | N.D. |
| C_0018 | 1-Methyl-2-pyrrolidone | N.D. | N.D. | N.D. | N.D. | N.D. | N.D. |
| C_0094 | 1-Methyl-4-imidazoleacetic acid | N.D. | N.D. | N.D. | N.D. | N.D. | N.D. |
| C_0218 | 1-Methyladenosine | 2.7 | N.D. | N.D. | N.D. | 3.1 | 2.2 |
| C_0060 | 1-Methylhistamine | N.D. | N.D. | N.D. | N.D. | N.D. | N.D. |
| C_0088 | 1-Methylnicotinamide 11-Aminoundecanoic | N.D. | N.D. | N.D. | N.D. | 5.5 | 2.8 |
| C_0175 | acid | N.D. | N.D. | N.D. | N.D. | N.D. | N.D. |
| C_0194 | 2'-Deoxycytidine | 12 | 9.4 | 9.6 | 6.6 | 11 | 7.1 |
| A_0145 | 2.3-Diphosphoglyceric acid | 29 | 22 | 24 | 23 | 26 | 23 |
| C_0044 | 2.4-Diaminobutyric acid | N.D. | N.D. | N.D. | N.D. | N.D. | N.D. |
| A_0055 | 2,5-Dihydroxybenzoic acid 2-Amino-2-methyl- | N.D. | N.D. | N.D. | N.D. | N.D. | N.D. |
| C_0031 | 1,3-propanediol | N.D. | N.D. | N.D. | ND. | ND. | ND. |
| C_0125 | 2-Aminoadipic acid | 49 | 64 | 49 | 131 | 280 | 167 |
| C_0024 | 2-Aminobutyric acid | N.D. | N.D. | N.D. | N.D. | N.D. | N.D. |
| A_0024 | 2-Aminoethylphosphonic acid | N.D. | N.D. | N.D. | N.D. | N.D. | N.D. |
| C_0027 | 2-Aminoisobutyric acid | N.D. | N.D. | N.D. | N.D. | N.D. | N.D. |
| A.0035 | 2-Hydroxy-4-methylvaleric acid | N.D. | N.D. | N.D. | N.D. | N.D. | N.D. |
| A_0011 | 2-Hydroxybutyric acid | N.D. | N.D. | N.D. | N.D. | N.D. | N.D . |
| A_0046 | 2-Hydroxyglutaric acid | ND. | N.D. | N.D. | N.D. | 17 | 13 |
| A_0091 | 2-Isopropylmalic acid | N.D. | N.D. | N.D. | N.D. | N.D. | N.D. |
| C_0047 | 2-Methylserine | N.D. | N.D. | N.D. | N.D. | N.D. | N.D. |
| A_0059 | 2-Oxoadipic acid | N.D. | N.D. | N.D. | N.D. | N.D. | N.D. |
| A_0045 | 2-Oxoglutaric acid | N.D. | N.D. | N.D. | ND. | 50 | N.D. |
| A_0017 | 2-Oxoisovaleric acid | N.D. | N.D. | N.D. | N.D. | N.D. | N.D. |
| C_0052 | 2-Phenylethylamine | N.D. | N.D. | N.D. | N.D. | N.D. | N.D. |

**[Table 8-2]**

| ID | Metabolite | Concentration (pmol/10⁸ cells) | | | | | |
|---|---|---|---|---|---|---|---|
| | | 30-3-13 | 30-3-14 | 30-3-15 | 33-72-16 | 33-72-17 | 33-72-18 |
| A_0098 | 2-Phosphoglyceric acid | N.D. | 16 | N.D. | N.D. | 12 | N.D. |
| A_0186 | 3',5'-ADP | N.D. | N.D. | N.D. | N.D. | N.D. | N.D. |
| A_0164 | 3'-AMP | N.D. | N.D. | N.D. | N.D. | N.D. | N.D. |
| A_0211 | 3'-Dephospho CoA | N.D. | N.D. | ND. | N.D. | N.D. | N.D. |
| A_0068 | 3-(2-Hydroxyphenyl)propionic acid | N.D. | N.D. | N.D. | N.D. | N.D. | N.D. |
| A_0067 | 3-(4-Hydroxyphenyl)propionic acid | N.D. | N.D. | N.D. | N.D. | N.D. | N.D. |
| C_0022 | 3-Aminobutyric acid | N.D. | N.D. | N.D. | N.D. | N.D. | N.D. |
| C_0025 | 3-Aminoisobutyric acid | N.D. | N.D. | N.D. | N.D. | N.D. | N.D. |
| C_0016 | 3-Aminopropane-1,2-diol | N.D. | N.D. | ND. | N.D. | N.D. | N.D. |
| A_0061 | 3-Hydroxy-3-methylglutaric acid | N.D | N.D. | N.D. | N.D. | N.D. | N.D. |
| C_0115 | 3-Hydroxyanthranilic acid | N.D. | N.D. | N.D. | N.D. | N.D. | N.D. |
| A_0012 | 3-Hydroxybutyric acid | N.D. | N.D. | N.D. | N.D. | N.D. | N.D. |
| C_0192 | 3-Hydroxykynurenine | N.D. | N.D. | N.D. | N.D. | N.D. | N.D. |
| A_0008 | 3-Hydroxypropionic acid | N.D. | N.D. | ND. | N.D. | N.D. | N.D. |
| A_0125 | 3-Indoxylsulfuric acid | N.D. | N.D. | N.D. | N.D. | N.D. | N.D. |
| C_0136 | 3-Methoxytyramine | N.D. | N.D. | N.D. | N.D. | N.D. | N.D. |
| C_0184 | 3-Methoxytyrosine | N.D. | N.D. | N.D. | N.D. | N.D. | N.D. |
| C_0109 | 3-Methyladenine | N.D. | N.D. | N.D. | ND. | N.D. | N.D. |
| A_0069 | 3-Phenyllactic acid | N.D. | N.D. | N.D. | N.D. | N.D. | N.D. |
| A_0051 | 3-Phenylpropionic acid | N.D. | N.D. | N.D. | N.D. | N.D. | 11 |
| A_0099 | 3-Phosphoglyceric acid | N.D. | 77 | 87 | 43 | 59 | 45 |
| A_0034 | 3-Ureidopropionic acid | N.D. | N.D. | N.D. | N.D. | N.D. | N.D. |
| C_0117 | 4-(*β-*Acetylaminoethyl)imidazol e | N.D. | N.D. | N.D. | N.D. | N.D. | N.D. |
| A_0044 | 4-Acetamidobutanoic acid | N.D. | N.D. | N.D. | N.D. | N.D. | N.D. |
| C_0046 | 4-Amino-3-hydroxybutyric acid | N.D. | N.D. | N D. | N D. | N.D. | N.D. |

**[Table 8-3]**

| ID | Metabolite | Concentration (pmol/10⁸ cells) | | | | | |
|---|---|---|---|---|---|---|---|
| | | 30--3--13 | 30-3-14 | 30-3-15 | 33-72-16 | 33-72-17 | 33-72-18 |
| C_0169 | 4-Aminohippuric acid | N.D. | N.D. | N.D. | N.D. | N.D. | N.D. |
| C_0098 | 4-Guanidinobutyric acid | 9.1 | 9.3 | 7.9 | 7.5 | 12 | 10 |
| A_0018 | 4-Oxovaleric acid | N.D. | N.D. | N.D. | N.D. | N.D. | N.D. |
| A_0096 | 4-Pyridoxic acid | N.D. | N.D. | N.D. | N.D. | N.D. | N.D. |
| C_0224 | 5'-Deoxy-5'-methylthioadenosine 5- Amino-4-oxovaleric | N.D. | N.D. | 3.6 | 3.1 | 4.9 | 3.7 |
| C_0071 | acid | N.D. | N.D. | N.D. | N.D. | N.D. | N.D. |
| C_0041 | 5-Aminovaleric acid 5-Hydroxyindoleacetic | N.D. | N.D. | N.D. | N.D. | N.D. | N.D. |
| A_0106 | acid | N.D. | N.D. | N.D. | N.D. | N.D. | N.D. |
| C_0128 | 5-Hydroxylysine | N.D. | N.D. | N.D. | N.D. | N.D. | N.D. |
| C_0188 | 5-Hydroxytryptophan | N.D. | N.D. | N.D. | N.D. | N.D. | N.D. |
| C_0203 | 5-Methyl-2'-deoxycytidine | N.D. | N.D. | N.D. | N.D. | N.D. | N.D. |
| C_0059 | 5-Methylcytosine | N.D. | N.D. | N.D. | N.D. | N.D. | N.D. |
| A_0029 | 5-Oxolvexanoic acid | N.D. | N.D. | N.D. | N.D. | N.D. | N.D. |
| A_0026 | 5-Oxoproline | 432 | 1,439 | 540 | 377 | 474 | 356 |
| C_0077 | 6-Aminohexanoic acid | N.D. | N.D. | N.D. | N.D. | N D. | N.D. |
| A_0147 | 6-Phosphogluconic acid | N.D. | N.D. | N.D. | 4.8 | N.D. | N.D. |
| C_0132 | 7-Methylguanine | N.D. | N.D. | N.D. | ND. | N.D. | N.D. |
| A_0178 | Acetyl CoA_divalent | N.D. | 7.7 | 3.6 | 3.7 | 4.8 | 4.5 |
| C_0085 | Adenine | N.D. | N.D. | N.D. | 2.8 | 3.1 | 2.1 |
| C_0213 | Adenosine | 7.3 | 8.3 | 5.1 | 5.3 | 13 | 7.1 |
| A_0191 | Adenylosuccinic acid | N.D. | N.D. | N.D. | 7.0 | 12 | 7.2 |
| C_0177 | ADMA | 7.1 | 6.2 | 5.7 | 3.5 | 5.5 | 3.8 |
| A_0185 | ADP | 299 | 370 | 438 | 424 | 754 | 454 |
| A_0201 | ADP-ribose | N.D. | N.D. | N.D. | N.D. | 1.8 | N.D. |
| C_0156 | Adrenaline | N.D. | N.D. | N.D. | N.D. | N.D. | N.D. |
| C_0070 | Agmatine | N.D. | N.D. | N.D. | N.D. | N.D. | N.D. |
| C_0013 | Ala | 1,062 | 1,396 | 1,108 | 583 | 976 | 644 |
| C_0122 | Ala-Ala | N.D. | N.D. | N.D. | ND. | N.D. | N.D. |
| A_0090 | Allantoic acid | N.D. | N.D. | N.D. | N.D. | N.D. | N.D. |
| C_0120 | Allantoin | N.D. | N.D. | N.D. | N.D. | N.D. | N.D. |

**[Table 8-4]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0004 | Aminoacetone | N.D. | N.D. | N.D. | N.D. | N.D. | N.D. |
| A_0165 | AMP | 125 | 158 | 135 | 211 | 436 | 210 |
| C_0050 | Anserine_divalent | N.D. | N.D. | N.D. | N.D. | N.D. | N.D. |
| C_0090 | Anthranilic acid | N.D. | N.D. | N.D. | ND. | N.D. | N.D. |
| C_0144 | Arg | 1,907 | 3,161 | 1,896 | 1,483 | 2,042 | 1,549 |
| C_0225 | Arg-Glu | N.D. | N.D. | 4.4 | 3.5 | 6.0 | 3.9 |
| C_0176 | Arginine ethyl ester | N.D. | N.D. | N.D. | N.D. | N.D. | N.D. |
| C_0223 | Argininosuccinic acid | 40 | 32 | 33 | 34 | 42 | 36 |
| A_0135 | Ascorbate 2-phosphate | 102 | 570 | 117 | 54 | 77 | 57 |
| A_0134 | Ascorbate 2-sulfate | ND. | N.D. | N.D. | N.D. | N.D. | N.D. |
| C_0079 | Asn | 615 | 949 | 620 | 353 | 620 | 403 |
| C_0082 | Asp | 1,326 | 1,488 | 1,155 | 743 | 1,257 | 756 |
| A_0198 | ATP | 1,895 | 2.415 | 2,096 | 1,424 | 1,988 | 1,689 |
| A_0103 | Azelaic acid | N.D. | N.D. | N.D. | N.D. | N.D. | N.D. |
| A_0023 | Benzoic acid | N.D. | N.D. | N.D. | N.D. | N.D. | N.D. |
| A_0050 | Benzoylformic acid | N.D. | N.D. | N.D. | N.D. | N.D. | N.D. |
| C_0043 | Betaine | ND. | N.D. | N.D. | N.D. | 32 | N.D. |
| C_0049 | Betaine aldehyde+H₂O | N.D. | N.D. | N.D. | N.D. | N.D. | N.D. |
| C_0121 | Betonicine | N.D. | N.D. | N.D. | N.D. | N.D. | N.D. |
| C_0201 | Biopterin | N.D. | N.D. | N.D. | N.D. | N.D. | N.D. |
| A_0132 | Biotin | 69 | 70 | 79 | 55 | 81 | 57 |
| C_0197 | Butyrylcarnitine | N.D. | N.D. | N.D. | N.D. | N.D. | N.D. |
| C_0020 | Cadaverine | N.D. | N.D. | N.D. | N.D. | N.D. | N.D. |
| C_0170 | Caffeine | N.D. | N.D. | N.D. | N.D. | N.D. | N.D. |
| A_0160 | cAMP | N.D. | N.D. | N.D. | N.D. | N.D. | N.D. |
| C_0182 | Carboxymethyllysine | N.D. | N.D. | N.D. | N.D. | N.D. | N.D. |
| C_0127 | Carnitine | ND. | N.D | ND. | N.D. | N.D. | N.D |
| C_0193 | Carnosine | ND. | N.D. | N.D. | N.D. | N.D. | N.D. |
| A.0176 | CDP | 32 | 43 | 50 | 45 | 84 | 44 |
| A_0196 | CDP-choline | 75 | 86 | 81 | 54 | 88 | 53 |

**[Table 8-5]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| A_0163 | cGMP | N.D. | N.D. | N.D. | N.D. | N.D. | N.D. |
| C_0029 | Choline | 179 | 383 | 172 | 165 | 274 | 197 |
| C_0072 | *cis*-4-Hydroxyproline | N.D. | N.D. | N.D. | N.D. | N.D. | N.D. |
| A_0082 | *cis*-Aconitic acid | N.D. | N.D. | N.D. | N.D. | N.D. | N.D. |
| A_0027 | Citraconic acid | N.D. | N.D. | N.D. | N.D. | N.D. | N.D. |
| A_0047 | Citramalic acid | N.D. | N.D. | N.D. | N.D. | N.D. | N.D. |
| A_0108 | Citric acid | 294 | 288 | 275 | 234 | 315 | 267 |
| C_0148 | Citrulline | N.D. | N.D. | N.D. | N.D. | N.D. | N.D. |
| A_0157 | CMP | 27 | 32 | 23 | 32 | 67 | 36 |
| A_0172 | CoA_divalant | N.D. | N.D. | M.D. | 1.4 | 2.0 | N.D. |
| C_0074 | Creatine | 38 | 42 | 52 | 174 | 293 | 222 |
| C_0037 | Creatinine | N.D. | N.D. | N.D. | N.D. | N.D. | N.D. |
| A_0194 | CTP | 241 | 323 | 267 | 129 | 208 | 160 |
| C_0051 | Cys | 26 | 51 | 20 | 6.9 | 12 | 13 |
| C_0150 | Cys-Gly | N.D. | N.D. | N.D. | N.D. | N.D. | N.D. |
| C_0191 | Cystathionine | 165 | 170 | 172 | 141 | 246 | 149 |
| A_0076 | Cysteic acid | N.D. | N.D. | N.D. | N.D. | N.D. | N.D. |
| C 0237 | Cysteine glutathione disulfide | N.D. | 163 | N.D. | N.D. | N.D. | 7.1 |
| C_0202 | Cystine | N.D. | 14 | N.D. | ND. | N.D. | N.D. |
| C_0205 | Cytidine | 13 | 17 | 13 | 10 | 18 | 11 |
| C_0034 | Cytosine | N.D. | N.D. | N.D. | N.D. | N.D. | N.D. |
| A_0197 | dATP | N.D. | 5.6 | N.D. | 3.2 | 4.9 | 4.4 |
| A_0192 | dCTP | N.D. | N.D. | N.D. | N.D. | N.D. | N.D. |
| A_0080 | Decanoic acid | 38 | 60 | 52 | 26 | 15 | 19 |
| A_0199 | dGTP | N.D. | N.D. | N.D. | N.D. | N.D. | N.D. |
| A_0170 | Digalacturonic acid | N.D. | N.D. | N.D. | N.D. | N.D. | N.D. |
| A_0077 | Dihydroxyacetone phosphate | 220 | 263 | 165 | 267 | 382 | 267 |
| C_0173 | DOPA | N.D. | N.D. | N.D. | N.D. | N.D. | N.D. |
| C_0116 | Dopamine | N.D. | N.D. | N.D. | N.D. | N.D. | N.D. |
| A_0175 | dTDP | N.D. | N.D. | N.D. | 4.5 | 7.5 | N.D. |

**[Table 8-6]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72--18 |
| A_0202 | dTDP-glucose | N.D. | N.D. | N.D. | N.D. | N.D. | N.D. |
| A_0155 | dTMP | 6.1 | 4.5 | N.D. | 8.9 | 12 | 8.0 |
| A_0193 | dTTP | 16 | 19 | 18 | 12 | 14 | 11 |
| C_0096 | Ectoine | ND. | N.D. | N.D. | N.D. | N.D. | N.D. |
| A_0116 | Erythrose 4-phosphate | N.D. | N.D. | N.D. | N.D. | N.D. | N.D. |
| C_0003 | Ethanolamine | 185 | 287 | 59 | 47 | 239 | 57 |
| A_0042 | Ethanolamine phosphate | 4,088 | 4,522 | 4,546 | 3,834 | 5,007 | 3,728 |
| A_0114 | Ferulic acid | N.D. | N.D. | N.D. | N.D. | N.D. | N.D. |
| A_0190 | FMN | N.D. | N.D. | N.D. | N.D. | N.D. | N.D. |
| A_0065 | Formylanthranilic acid | N.D | N.D. | N.D. | N.D. | N.D. | N.D. |
| A_0162 | Fructose 1,6-diphosphate | 666 | 847 | 585 | 1,161 | 1,764 | 1,060 |
| A.0140 | Fructose 6-phosphate | 20 | 26 | 19 | 30 | 52 | 28 |
| A_0016 | Fumaric acid | 151 | 129 | 134 | 108 | 112 | 96 |
| C_0028 | GABA | N.D. | N.D. | N.D. | ND. | N.D. | N.D. |
| A_0146 | Galacturonate 1-phosphate | N.D. | N.D. | N.D. | N.D. | N.D. | N.D. |
| A_0188 | GDP | 80 | 101 | 111 | 110 | 191 | 110 |
| C_0101 | Gln | 6,634 | 12,814 | 6,857 | 4.165 | 6.354 | 4.558 |
| C_0103 | Glu | 6,838 | 7,961 | 7,215 | 3.356 | 5,754 | 3,775 |
| C_0216 | Glu-Glu | 16 | 19 | 16 | 15 | 20 | 15 |
| A_0116 | Gluconic acid | N.D. | N.D. | N.D. | ND. | N.D. | N.D. |
| A_0136 | Glucosamine 6-phosphate | N.D. | N.D. | N.D. | N.D. | N.D. | N.D. |
| C_0171 | Glucosaminic acid | N.D. | N.D. | N.D. | N.D. | N.D. | N.D. |
| A_0138 | Glucose 1-phosphate | 73 | 69 | 72 | 54 | 89 | 54 |
| A_0139 | Glucose 6-phosphate | 36 | 36 | 34 | 61 | 81 | 51 |
| A_0033 | Glutaric acid | N.D. | N.D. | N.D. | N.D. | N.D. | N.D. |
| C_0227 | Glutathione (GSH) | 385 | 191 | 339 | 114 | 205 | 130 |
| C_0226 | Glutathione (GSSG) divalent | 319 | 440 | 458 | 212 | 364 | 272 |
| C_0008 | Gly | 2,452 | 3,323 | 2,593 | 1.630 | 2,604 | 1,766 |
| C_0167 | Gly-Asp | 17 | 15 | 15 | 13 | 20 | 14 |
| C_0078 | Gly-Gly | 18 | 19 | 20 | 14 | 21 | 17 |

**[Table 8-7]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0161 | Gly-Leu | N.D. | N.D. | N.D. | N.D. | N.D. | N.D. |
| A_0078 | Glyceraldehyde 3-phosphate | 99 | 91 | 82 | 120 | 150 | 136 |
| A_0013 | Glyceric acid | N.D. | N.D. | N.D. | N.D. | N.D. | N.D. |
| A_0079 | Glycerol 3-phosphate | 61 | 62 | 55 | 37 | 69 | 36 |
| C_0211 | Glycerophosphocholine | 96 | 102 | 103 | 64 | 129 | 76 |
| A_0003 | Glycolic acid | N.D. | N.D. | N.D. | N.D. | N.D. | N.D. |
| A_0001 | Glyoxylic acid | N.D. | N.D. | N.D. | N.D. | N.D | N.D. |
| A_0167 | GMP | 33 | 51 | 33 | 47 | 80 | 45 |
| A.0200 | GTP | 519 | 665 | 559 | 375 | 591 | 431 |
| C_0146 | Guanidinosuccinic acid | N.D. | N.D. | N.D. | N.D. | N.D. | N.D. |
| C_0040 | Guanidoacetic acid | N.D. | N.D. | N.D. | N.D. | N.D. | N.D. |
| C_0111 | Guanine | 13 | N.D. | 17 | N.D. | 7.5 | N.D. |
| C_0219 | Guanosine | 17 | 20 | 16 | 13 | 27 | 13 |
| A_0030 | Heptanoic acid | N.D. | N.D. | 7.9 | 4.6 | 4.3 | 5.6 |
| A_0093 | Hippuric acid | N.D. | N.D. | N.D. | N.D. | N.D. | N.D. |
| C_0118 | His | 306 | 663 | 292 | 157 | 270 | 186 |
| C_0220 | His-Glu | N.D. | N.D. | N.D. | N.D. | N.D. | 1.9 |
| C_0035 | Histamine | N.D. | N.D. | N.D. | N.D. | N.D. | N.D. |
| C_0095 | Histidinol | N.D | N.D. | N.D | N.D. | N.D. | N.D. |
| C_0163 | Homoarginine | ND. | N.D. | N.D. | N.D. | N.D. | N.D. |
| C_0166 | Homocitrulline | N.D. | N.D. | N.D. | N.D. | N.D. | N.D. |
| A_0095 | Homocysteic acid | N.D. | N.D. | N.D. | N.D. | N.D. | N.D. |
| C_0083 | Homocysteine | N.D. | N.D. | N.D. | N.D. | N.D. | N.D. |
| C_0048 | Homoserine | N.D. | N.D, | N.D. | N.D. | N.D. | N.D. |
| A_0094 | Homovanillic acid | N.D. | N.D. | N.D. | N.D. | N.D. | N.D. |
| C_0073 | Hydroxyproline | N.D. | N.D. | N.D. | 9.4 | 12 | 9.4 |
| C_0033 | Hypotaurine | N.D. | N.D. | N.D. | N.D. | N.D. | N.D. |
| C_0086 | Hypoxanthine | 136 | 153 | 135 | 30 | 39 | 38 |
| A_0187 | IDP | N.D. | ND. | N.D. | N.D. | N.D. | N.D. |
| C_0076 | lle | 516 | 1,361 | 560 | 311 | 494 | 344 |

**[Table 8-8]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0061 | Imidazole-4-acetic acid | N.D. | N.D . | N.D. | N.D. | N.D. | N.D. |
| C_0119 | Imidazolelactic acid | N.D. | N.D. | N.D. | N.D. | N.D. | N.D. |
| A_0166 | IMP | 349 | 261 | 277 | 579 | 679 | 496 |
| C_0141 | Indole-3-acetamide | N.D. | N.D. | N.D. | N.D. | ND. | N.D. |
| A_0087 | Indole-3-acetic acid | N.D. | N.D. | N.D. | N.D. | N.D. | N.D. |
| C_0126 | Indole-3-ethanol | N.D. | N.D. | N.D. | N.D. | N.D. | N.D. |
| C..0215 | Inosine | 150 | 126 | 125 | 133 | 193 | 133 |
| A_0025 | Isethionic acid | N.D. | N.D. | N.D. | N.D. | 5.1 | 3.5 |
| C_0011 | Isoamylamine | N.D. | N.D. | N.D. | N.D. | N.D. | N.D. |
| C_0006 | Isobutylamine | N.D. | N.D | N.D. | N.D. | N.D | N.D. |
| C_0196 | Isobutyrylcarnitine | N.D. | N.D. | N.D. | N.D. | N.D. | N.D. |
| A_0110 | Isocitric acid | N.D. | N.D. | N.D. | N.D. | N.D. | N.D. |
| C_0106 | Isoglutamic acid | N.D. | N.D. | N.D. | N.D. | N.D. | N.D. |
| C_0053 | Isoniootinamide | ND. | N.D. | N.D. | N.D. | N.D. | N.D. |
| C_0055 | Isonicotinic acid | N.D. | N.D. | N.D. | N.D. | N.D. | N.D. |
| C_0209 | Isovalerylcarnitine | N.D. | N.D. | N.D. | N.D. | N.D. | N.D. |
| A_0104 | Kynurenic acid | N.D. | N.D. | N.D. | N.D. | N.D. | N.D. |
| C_0183 | Kynurenine | 21 | 16 | 20 | 25 | 37 | 26 |
| A_0007 | Lactic acid | 6,898 | 8.099 | 7,767 | 3,483 | 4,366 | 3,537 |
| C_0075 | Leu | 599 | 1,539 | 623 | 360 | 602 | 417 |
| C_0236 | Leu-Leu-Tyr | N.D. | N.D. | N.D | N.D. | N.D. | N.D. |
| C_0102 | Lys | 1,830 | 2.901 | 1,866 | 1,319 | 1,800 | 1,391 |
| A_0039 | *m*-Hydroxybenzoic acid | N.D. | N.D. | N.D. | N.D. | N.D. | N.D. |
| A_0015 | Maleic acid | N.D. | N.D. | N.D. | N.D. | N.D. | N.D. |
| A_0036 | Malic acid | 484 | 397 | 398 | 374 | 429 | 351 |
| A_0183 | Malonyl CoA_divalent | N.D. | N.D. | N.D. | N.D. | N.D. | N.D. |
| C_0198 | Melatonin | N.D. | N.D. | N.D. | N.D. | N.D. | N.D. |
| C_0108 | Met | 134 | 322 | 155 | 80 | 129 | 93 |
| C_0131 | Methionine sulfoxide | N.D. | 30 | 15 | 8.7 | 12 | 10 |
| C_0005 | Methylguanidine | N.D. | N.D. | N.D. | N.D. | N.D. | N.D. |

**[Table 8-9]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0068 | Mevalolactone | N.D. | N.D. | N.D. | N.D. | N.D. | N.D. |
| A_0048 | Mevalonic acid | N.D. | N.D. | N.D. | N.D. | N.D. | N.D. |
| A_0123 | Mucic acid | 8.6 | 17 | 17 | 9.5 | 15 | 8.5 |
| C_0038 | Muscimol | N.D. | N.D. | N.D. | N.D. | N.D. | N.D. |
| A_0137 | *myo*-Inositol 2-phosphate | N.D. | N.D. | N.D. | 8.7 | 6.2 | 7.2 |
| C_0023 | *N,N-*Dimethylglycine | N.D. | N.D. | N.D. | N.D. | N.D. | N.D. |
| C_0158 | *N,N*-Dimethylhistidine | N.D. | N.D. | N.D. | N.D. | N.D. | N.D. |
| A_0032 | *N*-Acetyl-*β*-alanine | N.D. | N.D. | N.D. | ND. | N.D. | N.D. |
| A_0031 | *N*-Acatylalanine | 14 | 14 | 15 | 12 | 18 | 16 |
| A_0084 | *N*-Acetylasparagine | N.D. | N.D. | N.D. | N.D. | N.D. | N.D. |
| A_0086 | *N*-Acetylaspartic acid | 295 | 338 | 331 | 359 | 523 | 388 |
| C_0190 | *N*-Acetylglucosamine | N.D. | N.D. | N.D. | N.D. | N.D. | N.D. |
| A_0151 | *N*-Acetylglucosamine 1-phosphate | 14 | 14 | 17 | 15 | 21 | 17 |
| A_0152 | *N*-Acetylglucosime 6-phosphate | 5.3 | 5.2 | 54 | N.D. | 4.5 | 3.1 |
| A_0105 | *N*-Acetylglutamic acid | N.D. | N.D. | N.D. | 6.8 | 11 | 8.3 |
| A_0102 | *N*-Acetylglutamine | N.D. | N.D. | N.D. | N.D. | N.D. | N.D. |
| A_0020 | *N*-Acetylglycine | N.D. | N.D. | N.D. | N.D. | N.D. | N.D. |
| C_0174 | *N-*Acetylhistidine | N.D. | N.D. | N.D. | N.D. | N.D. | N.D. |
| A_0081 | *N-*Acetylleucine | N.D. | N.D. | N.D. | N.D. | N.D. | N.D. |
| A_0107 | *N*-Acetylmethionine | N.D. | N.D. | N.D. | N.D. | 1.2 | N.D. |
| A_0153 | *N*-Acetylneuraminic acid | 10 | 13 | 14 | 13 | 16 | 15 |
| C_0142 | *N*-Acetylornithine | 11 | 14 | 10 | 9.1 | 16 | 11 |
| C_0089 | *N*-Acetylputrescine | N.D. | N.D. | N.D. | 2.5 | 4.3 | 2.3 |
| A_0133 | *N*-Acetyltryptophan | N.D. | N.D. | N.D. | N.D. | N.D. | N.D. |
| A_0089 | *N*-Carbamoylaspartic acid | N.D. | N.D. | N.D. | N.D. | N.D. | N.D. |
| C_0021 | *N*-Ethylglycine | N.D. | N.D. | N.D. | N.D. | N.D. | N.D. |
| A_0092 | *N*-Formylmethionine | N.D. | N.D. | N.D. | ND. | N.D. | ND. |
| A_0159 | *N*-Glycolylneuraminic acid | N.D. | N.D. | N.D. | ND. | N.D. | N.D. |
| C_0026 | *N*-Methylelanine | N.D. | N.D. | N.D. | N.D. | N.D. | N.D. |
| C_0112 | *N-*Methylanthranilic acid | N.D. | N.D. | N.D. | ND. | N.D. | N.D. |

**[Table 8-10]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0124 | *N*-Methylglutamic acid | N.D. | N.D. | N.D. | N.D. | N.D. | N.D. |
| C_0087 | *N-*Methylnicotinamide | N.D. | N.D. | N.D. | N.D. | N.D. | N.D. |
| C_0065 | *N*-Methylproline | N.D. | N.D. | N.D. | N.D. | N.D. | N.D. |
| C_0019 | *N*-Methylputrescine | N.D. | N.D. | N.D. | N.D. | N.D. | N.D. |
| C_0168 | *N*-Methylserotonin | N.D. | N.D. | N.D. | N.D. | N.D. | N.D. |
| C_0145 | *N*-Methyltryptamine | N.D. | N.D. | N.D. | N.D. | N.D. | N.D. |
| C_0186 | *N-*Methyltryptophan | N.D. | N.D. | N.D. | N.D. | N.D. | N.D. |
| C_0208 | *N,N*¹-Diethylnorspermine | N.D. | N.D. | N.D. | N.D. | N.D. | N.D. |
| C_0067 | *N,N*²-Diethylspermine_divalent | N.D. | N.D. | N.D. | N.D. | N.D. | N.D. |
| C_0195 | *N,N*³-Diacetylspermidine | N.D. | N.D. | N.D. | N.D. | N.D. | N.D. |
| C_0159 | *N*-Acetylspermidine | N.D. | N.D. | N,D. | N.D, | N.D. | N.D, |
| C_0207 | *N*-Acetylspermine | N.D, | N.D. | N.D. | N.D. | N.D. | N.D. |
| C_0222 | *N-*Glutathionylspermidine disulfide trivalent | N.D. | N.D. | N.D. | N.D. | N.D. | N.D. |
| C_0114 | *N*-Methyl-4-pyridone-5-carboxamide | N.D. | N.D. | N.D. | N.D. | N.D. | N.D. |
| A_0144 | *N-*Phenylacetylglutamine | N.D. | N.D. | N.D. | N.D. | N.D. | N.D. |
| C_0143 | *N⁶*-Ethylgiutamine | N.D. | N.D. | N.D. | ND. | N.D. | ND. |
| C_0165 | *N⁶,N⁶,N⁶*-Trimethyllysine | 16 | 16 | 17 | 24 | 38 | 27 |
| C_0162 | *N*⁶-Acetyllysine | N.D. | N.D. | N.D. | 9.9 | 16 | 12 |
| C_0123 | *N⁶-*Methyllysine | 11 | 17 | 9.8 | 7.3 | 7.2 | 6.1 |
| 0.0160 | *N*⁶-Acetytspermidine | N.D. | N.D. | N.D. | N.D. | N.D. | N.D. |
| A_0209 | NAD⁺ | 212 | 212 | 225 | 219 | 281 | 205 |
| A_0212 | NADP* | N.D. | 7.7 | 5.0 | 3.6 | 5.4 | 3.2 |
| C_0054 | Nicotinamide | 17 | 48 | 22 | 10 | 14 | 12 |
| C..0129 | Nicotine | N.D. | N.D. | N.D. | N.D. | N.D. | N.D. |
| C_0056 | Nicotinic acid | N.D. | N.D. | N.D. | N.D. | N.D. | N.D. |

**[Table 8-11]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0230 | NMN | N.D. | N.D. | N.D. | N.D. | N.D. | N.D. |
| C_0140 | Noradrenaline | N.D. | N.D. | N.D. | N.D. | N.D. | N.D. |
| C_0157 | Normetanephrine | N.D. | N.D. | N.D. | N.D. | N.D. | N.D. |
| C_0164 | *N*_{w}-Methylarginine | N.D. | N.D. | N.D. | N.D. | N.D. | N.D. |
| C_0180 | *O*-Acetylcarnitine | N.D. | N.D. | N,D. | N.D. | N.D, | N.D. |
| C_0105 | *O*-Acetylserine | N.D. | N.D. | N.D. | N.D. | N.D. | N.D. |
| A_0063 | o-Coumaric acid | N.D. | N.D. | N.D. | N.D. | N.D. | N.D. |
| A_0040 | *o*-Hydroxybenzoic acid | N.D. | N.D. | N.D. | N.D. | N.D. | N.D. |
| A_0115 | *o*-Hydroxyhippuric acid | N.D. | N.D. | N.D. | N.D. | N.D. | N.D. |
| A_0097 | *O*-Phosphoserine | 51 | 55 | 55 | 28 | 59 | 26 |
| A_0126 | *O*-Succinylhomoserine | N.D. | N.D. | N.D. | N.D. | N.D. | N.D. |
| C_0221 | Octancylcarnitine | N.D. | N.D. | N.D. | N.D. | N.D. | N.D. |
| C_0080 | Ornithine | 190 | 194 | 212 | 74 | 110 | 87 |
| A_0057 | Orotic acid | N.D. | N.D. | N.D. | N.D. | N.D. | N.D. |
| A_0006 | Oxamic acid | N.D. | N.D. | N.D. | N.D. | N.D. | N.D. |
| C_0091 | p-Aminobenzoic acid | N.D. | N.D. | N.D. | N.D. | N.D. | N.D. |
| A_0052 | p-Anisic acid | N.D. | N.D. | N.D. | N.D. | N.D. | N.D. |
| A_0064 | *p*-Coumaric acid | N.D. | N.D. | N.D. | N.D. | N.D. | N.D. |
| A_0041 | p-Hydroxybenzoic acid | N.D. | N.D. | N.D. | N.D. | N.D. | N.D. |
| A_0075 | p-Hydroxymandelic acid | N.D. | N.D. | N.D. | N.D. | N.D. | N.D. |
| A_0054 | p-Hydroxyphenylacetic acid | N.D. | N.D. | N.D. | N.D. | N.D. | N.D. |
| A_0127 | Pantothenic acid | 112 | 136 | 112 | 124 | 171 | 128 |
| C_0152 | Paraxanthine | N.D. | N.D. | N.D. | N.D. | N.D. | N.D. |
| A_0058 | Pelargonic acid | 42 | 47 | 51 | 32 | 24 | 28 |
| A_0070 | Perillic acid | N.D. | N.D. | N.D. | N.D. | N.D. | N.D. |
| C_0133 | Phe | 326 | 804 | 334 | 196 | 322 | 223 |
| A_0112 | Phenaceturic acid | N.D. | N.D. | N.D, | N.D. | N.D. | N.D. |
| A_0053 | Phenoxyacetic acid | N.D. | N.D. | N.D. | N.D. | N.D. | N.D. |
| A_0124 | Phosphocreatine | N.D. | 110 | 57 | 197 | 224 | 270 |
| A_0072 | Phosphoenolpyruvic acid | 44 | 46 | 44 | 18 | 24 | 22 |

**[Table 8-12]**

| ID | Metabolite | Concentration (pmol/10⁸ cells) | | | | | |
|---|---|---|---|---|---|---|---|
| | | 30-3-13 | 30-3-14 | 30-3-15 | 33-72-16 | 33-72-17 | 33-72-18 |
| A_0056 | Phosphoglycolic acid | N.D. | N.D. | N.D. | N.D. | N.D. | N.D. |
| C_0155 | Phosphorylcholine | 1,811 | 1,958 | 2,221 | 1,581 | 2,507 | 1.766 |
| C_0057 | Picolinic acid | N.D. | N.D. | N.D. | N.D. | N.D. | N.D. |
| A_0060 | Pimelic acid | N.D. | N.D. | N.D. | N.D. | N.D. | N.D. |
| C_0066 | Pipecolic acid | N.D. | N.D. | N.D. | N.D. | 4.0 | N.D. |
| C_0010 | Piperidine | N.D. | N.D. | ND. | N.D. | N.D. | N.D. |
| C_0039 | Pro | 1,339 | 1,706 | 1,364 | 860 | 1,330 | 917 |
| A_0002 | Propionic acid | N.D. | N.D. | N.D. | N.D. | N.D. | N.D. |
| A_0179 | Propionyl CoA_divalent | N.D. | N.D. | N.D. | N.D. | N.D. | N.D. |
| A_0173 | PRPP | N.D. | ND. | N.D. | 19 | 18 | 18 |
| C_0130 | Pterin | N.D. | N.D. | N.D. | N.D. | N.D. | N.D. |
| C_0012 | Putrescine | N.D. | 29 | N.D. | N.D. | 9.1 | N.D. |
| C_0135 | Pyridoxal | N.D. | ND. | 3.2 | N.D. | 3.2 | 2.9 |
| C_0137 | Pyridoxamine | N.D. | N.D. | N.D. | N.D. | N.D. | N.D. |
| C_0210 | Pyridoxamine 5'-phosphate | N.D. | ND. | 6.7 | 4.0 | 6.2 | 4.6 |
| C_0139 | Pyridoxine | 12 | 33 | 14 | 7.7 | 12 | 8.9 |
| A_0004 | Pyruvic acid | N.D. | N.D. | N.D. | N.D. | N.D. | N.D. |
| A_0111 | Quinic acid | N.D. | ND. | N.D. | N.D. | N.D. | N.D. |
| A_0071 | Quinolinic acid | N.D. | N.D. | N.D. | N.D. | N.D. | N.D. |
| C_0232 | Riboflavin | N.D. | N.D. | N.D. | N.D. | N.D. | N.D. |
| A_0130 | Ribose 5-phosphate | N.D. | 11 | N.D. | 6.8 | 8.5 | 6.7 |
| A_0154 | Ribulose 1,5-diphosphate | N.D. | ND. | N.D. | N.D. | N.D. | N.D. |
| A_0129 | Ribulose 5-phosphate | 32 | 22 | 27 | 24 | 28 | 26 |
| C_0234 | *S-*Adenosylhomocysteine | N.D. | N.D. | N.D. | N.D. | N.D. | N.D. |
| C_0235 | *S*-Adenosylmethionine | 61 | 61 | 65 | 54 | 76 | 56 |

**[Table 8-13]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0233 | *S*-Lactoylglutathione | N.D. | N.D. | N.D. | N.D. | N.D. | N.D. |
| C_0084 | *S*-Methylcysteine | N.D. | N.D. | N.D. | ND. | N.D. | N.D. |
| A_0120 | *S*-Sulfocysteine | N.D. | N.D. | N.D. | N.D. | N.D. | N.D. |
| C_0217 | Saccharopine | N.D. | N.D. | N.D. | N.D. | N.D. | N.D. |
| C_0014 | Sarcosine | N.D. | N.D. | N.D. | N.D. | N.D. | N.D. |
| C_0178 | SOMA | N.D. | N.D. | N.D. | N.D. | N.D. | N.D. |
| A_0121 | Sebacic acid | N.D. | N.D. | N.D. | N.D. | N.D. | N.D. |
| A_0150 | Sedoheptulose 7-phosphate | N.D. | N.D. | N.D. | 4.3 | 5.9 | N.D. |
| C_0030 | Ser | 1,397 | 2.194 | 1,498 | 974 | 1.525 | 1,064 |
| C_0199 | Ser-Glu | 9.0 | 8.6 | 7.4 | 6.1 | 9.7 | 5.9 |
| C_0149 | Serotonin | N.D. | N.D. | N.D. | N.D. | ND. | N.D |
| A_0083 | Shikimic acid | N.D. | N.D. | N.D. | N.D. | N.D. | N.D. |
| A_0143 | Sorbitol 6-phosphate | N.D. | N.D. | N.D. | N.D. | N.D. | N.D. |
| C_0100 | Spermidine | 7.4 | 19 | 7.3 | 7.6 | 14 | 8.6 |
| C_0179 | Spermine | N.D | 24 | N.D. | 15 | 30 | 14 |
| C_0097 | Stachydrine | N.D. | N.D. | N.D. | N.D. | N.D. | N.D. |
| A_0085 | Suberic acid | N.D. | N.D. | N.D. | N.D. | N.D. | N.D. |
| A_0021 | Succinic acid | N.D. | N.D. | N.D. | 46 | 48 | N.D. |
| A_0180 | Sucrose 6'-phosphate | N.D. | N.D. | N.D. | N.D. | N.D. | N.D. |
| A_0049 | Tartaric acid | N.D. | N.D. | N.D. | N.D. | N.D. | N.D. |
| C_0058 | Taurine | N.D. | N.D. | N.D. | N.D. | N.D. | N.D. |
| C_0134 | Taurocyamine | N.D. | N.D. | N.D. | N.D. | N.D. | N.D. |
| C_0212 | Thiamine | 45 | 123 | 41 | 26 | 32 | 26 |
| C_0231 | Thiamine phosphate | N.D. | N.D. | N.D. | N.D. | N.D. | N.D. |
| C_0081 | Thiaproline | 22 | 69 | 18 | 15 | 25 | 11 |
| C_0045 | Thr | 1,019 | 1,943 | 1,048 | 566 | 975 | 680 |
| C_0200 | Thr-Asp | 11 | 11 | 12 | 8.1 | 13 | 9.5 |
| C_0107 | *threo*-*β*-Methylaspartic acid | N.D. | N.D. | N.D. | N.D. | N.D. | N.D. |
| A_0037 | Threonic acid | 102 | 156 | 147 | 112 | 137 | 97 |
| C_0204 | Thymidine | N.D. | N.D. | N.D. | N.D. | N.D. | N.D. |

**[Table 8-14]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| C_0062 | Thymine | N.D. | N.D. | N.D. | N.D. | N.D. | N.D. |
| C_0187 | *trans*-Zeatin | N.D. | N.D. | N.D. | N.D. | N.D. | N.D. |
| A_0181 | Trehalose 6-phosphate | 287 | 279 | 184 | 173 | 206 | 186 |
| C_0089 | Trigonelline | N.D. | N.D. | N.D. | N.D. | N.D. | N.D. |
| C_0001 | Trimethylamine | N.D. | N.D. | N.D. | N.D. | N.D. | N.D. |
| C_0009 | Trimethylamine *N*-oxide | N.D. | N.D. | N.D. | N.D. | N.D. | N.D. |
| C_0181 | Trp | 54 | 143 | 59 | 28 | 44 | 33 |
| C_0154 | Tyr | 302 | 758 | 324 | 177 | 301 | 210 |
| C_0138 | Tyr-Arg_divalent | N.D. | N.D. | N.D. | N.D. | N.D. | N.D. |
| C_0228 | Tyr-Glu | N.D. | N.D. | N.D. | N.D. | N.D. | N.D. |
| C_0092 | Tyramine | N.D. | N.D. | N.D. | N.D, | N.D. | N.D. |
| C_0172 | Tyrosine methyl ester | N.D. | N.D. | N.D. | N.D. | N.D. | N.D. |
| A_0177 | UDP | 75 | 95 | 101 | 108 | 202 | 111 |
| A_0204 | UDP-glucuronic acid | 157 | 298 | 200 | 154 | 239 | 157 |
| A_0158 | UMP | 51 | 58 | 54 | 82 | 146 | 73 |
| C_0036 | Uracil | N.D. | N.D. | N.D. | N.D. | N.D. | N.D. |
| C_0002 | Urea | 1,040 | 953 | 896 | N.D. | 321 | 274 |
| A_0073 | Uric acid | N.D. | N.D. | N.D. | N.D. | N.D. | N.D. |
| C_0206 | Uridine | N.D. | N.D. | N.D. | N.D. | N.D. | N.D. |
| C_0093 | Urocanic acid | 5.6 | N.D. | N.D. | N.D. | N.D. | N.D. |
| A_0195 | UTP | 552 | 727 | 602 | 390 | 544 | 436 |
| C_0042 | Va! | 683 | 1,585 | 699 | 374 | 591 | 429 |
| A_0009 | Valeric acid | N.D. | N.D. | N.D. | N.D. | ND. | N.D. |
| A_0074 | Vanillic acid | N.D. | ND. | N.D. | N.D. | N.D. | N.D. |
| A_0117 | Vanillylmandelic acid | N.D. | N.D. | N.D. | N.D. | N.D. | N.D. |
| C_0113 | Xanthine | N.D. | N.D. | N.D. | N.D. | N.D. | N.D. |
| A_0149 | Xanthosine | N.D. | N.D. | N.D. | N.D. | ND. | N.D. |
| A_0122 | Xanthurenic acid | N.D. | N.D, | N.D. | N.D. | N.D. | N.D. |
| A_0168 | XMP | N.D. | N.D. | N.D. | ND. | N.D. | N.D. |
| C_0015 | *β*-Ala | 53 | 49 | 59 | 66 | 114 | 79 |
| C_0185 | *β*-Ala-Lys | N.D. | N.D. | N.D. | N.D. | N.D. | N.D. |
| C_0153 | *β*-Tyr | N.D. | N.D. | N.D. | N.D. | N.D. | N.D. |
| C_0099 | *γ*-Butyrobetaine | N.D. | N.D. | N.D. | 4.1 | 6.0 | 4.3 |

**[Table 9]**

| ID | Metabolite | Concentration (pmol/10⁶ cells) | | | | | |
|---|---|---|---|---|---|---|---|
| | | 30-3-13 | 30-3-14 | 30-3-15 | 33-72-16 | 33-72-17 | 33-72-18 |
| A_0078 | Glyceraldehyde 3-phosphate | 99 | 91 | 82 | 120 | 150 | 136 |
| A_0105 | *N*-Acetylglutamic acid | N.D. | N.D. | N.D. | 6.8 | 11 | 8.3 |
| A_0124 | Phosphocreatine | N.D. | 110 | 57 | 197 | 224 | 270 |
| A_0137 | *myo*-Inositol 2-phosphate | N.D. | N.D. | N.D. | 8.7 | 6.2 | 7.2 |
| A_0139 | Glucose 6-phosphate | 36 | 36 | 34 | 61 | 81 | 51 |
| A_0140 | Fructose 6-phosphate | 20 | 26 | 19 | 30 | 52 | 28 |
| A_0155 | dTMP | 6.1 | 4.5 | N.D. | 8.9 | 12 | 8.0 |
| A_0157 | CMP | 27 | 32 | 23 | 32 | 67 | 36 |
| A_0158 | UMP | 51 | 58 | 54 | 82 | 146 | 73 |
| A_0162 | Fructose 1,6-diphosphate | 666 | 847 | 585 | 1,161 | 1,764 | 1,060 |
| A_0165 | AMP | 125 | 158 | 135 | 211 | 436 | 210 |
| A_0166 | IMP | 349 | 261 | 277 | 579 | 679 | 496 |
| A_0167 | GMP | 33 | 51 | 33 | 47 | 80 | 45 |
| A_0173 | PRPP | N.D. | N.D. | N.D. | 19 | is | 18 |
| A_0177 | UDP | 75 | 95 | 101 | 108 | 202 | 111 |
| A_0185 | ADP | 299 | 370 | 438 | 424 | 754 | 454 |
| A_0191 | Adenylosuccinic acid | N.D. | N.D. | N.D. | 7.0 | 12 | 7.2 |
| C_0015 | *β*-Ala | 53 | 49 | 59 | 66 | 114 | 79 |
| C_0069 | *N*-Acetylputrescine | N.D. | N.D. | N.D. | 2.5 | 4.3 | 2.3 |
| C_0073 | Hydroxyproline | N.D. | N.D. | N.D. | 9.4 | 12 | 9.4 |
| C_0074 | Creatine | 38 | 42 | 52 | 174 | 293 | 222 |
| C_0085 | Adenine | N.D. | N.D. | N.D. | 2.8 | 3.1 | 2.1 |
| C_0099 | *γ*-Butyrobetaine | N.D. | N.D. | N.D. | 4.1 | 6.0 | 4.3 |
| C_0125 | 2-Aminoadipic acid | 49 | 64 | 49 | 131 | 280 | 167 |
| C_0162 | *N'*-Acetyllysine | N.D. | N.D. | N.D. | 9.9 | 16 | 12 |
| C_0165 | *N*',*N*',*N*'-Trimethyllysine | 16 | 16 | 17 | 24 | 38 | 27 |
| C_0183 | Kynurenine | 21 | 16 | 20 | 25 | 37 | 26 |

It has been confirmed that each human iPS cell culture produces such various metabolites as shown in Table 9.

### INDUSTRIAL APPLICABILITY

According to the method of culturing human induced pluripotent stem cells according to the present embodiment, it is possible to form a culture of human induced pluripotent stem cells which is suitable for the formation of cell aggregates having neuroepithelial cells that have budded layeredly.

## Claims

1. A method of culturing human induced pluripotent stem cells, comprising a step of inoculating human induced pluripotent stem cells in a culture medium at an inoculation density of 1.0 × 10⁴ to 1.0 × 10⁶ cells/cm² in a culture vessel and subjecting the human induced pluripotent stem cells to two-dimensional culturing.

2. The method of culturing human induced pluripotent stem cells according to Claim 1,
wherein the step of carrying out the two-dimensional culturing includes a step of carrying out culturing in a culture medium that contains a Rho kinase inhibitor.

3. The method of culturing human induced pluripotent stem cells according to Claim 1 or 2,
wherein the step of carrying out the two-dimensional culturing includes a step of carrying out culturing in a culture medium that does not contain a Rho kinase inhibitor.

4. The method of culturing human induced pluripotent stem cells according to any one of Claims 1 to 3,
wherein a confluency after the step of carrying out the two-dimensional culturing is 70% to 100% by area.

5. The method of culturing human induced pluripotent stem cells according to any one of Claims 1 to 4,
wherein the culture vessel is a culture vessel subjected to a surface treatment that improves cell adhesiveness.

6. A method of producing cerebral organoids, comprising:
a step 1 of culturing a culture of the human induced pluripotent stem cells obtained by the method of culturing human induced pluripotent stem cells according to any one of Claims 1 to 5 in a culture medium containing a BMP inhibitor and a transforming growth factor β (TGFβ) inhibitor to form cell aggregates;
a step 2 of culturing the cell aggregates in a culture medium containing a Wnt signal transduction pathway potentiator and an extracellular matrix; and
a step 3 of subjecting the culturing obtained in the step 2 to spinner culturing.

7. The method of producing cerebral organoids according to Claim 6,
wherein the spinner culturing in the step 3 is spinner culturing in a culture medium that does not contain the extracellular matrix.

8. A culture of human induced pluripotent stem cells, which produces three or more metabolites selected from the group consisting of metabolites shown in Table 1, where a production amount of each of the metabolites is within a range shown in Table 1,
**[Table 1]**
| Metabolite | Production amount (pmol/10⁶ cells) |
|---|---|
| Glyceraldehyde 3-phosphate | 100 to 200 |
| Phosphocreatine | 150 to 400 |
| thymidine monophosphate (dTMP) | 7 to 20 |
| cytidine monophosphate (CMP) | 25 to 80 |
| uridine monophosphate (UMP) | 50 to 200 |
| Fructose 1,6-diphosphate | 800 to 2,000 |
| Adenosine monophosphate (AMP) | 150 to 500 |
| Inosine monophosphate (IMP) | 400 to 800 |
| guanosine triphosphate (GMP) | 30 to 100 |
| Uridine diphosphate (UDP) | 80 to 250 |
| Adenosine diphosphate (ADP) | 350 to 900 |
| Adenylosuccinic acid | 1 to 30 |
| Hydroxyproline | 8 to 20 |
| Creatine | 100 to 400 |
| 2-Aminoadipic acid | 100 to 350 |
| N⁶-Acetyllysine | 8 to 20 |
| N⁶,N⁶,N⁶-Trimethyllysine | 20 to 45 |
| Kynurenine | 20 to 45 |

9. The culture of human induced pluripotent stem cells according to Claim 8,
wherein the metabolites include adenylosuccinic acid.

10. The culture of human induced pluripotent stem cells according to Claim 8 or 9,
wherein the metabolites include inosine monophosphate.

11. The culture of human induced pluripotent stem cells according to any one of Claims 8 to 10,
wherein the metabolites include adenosine monophosphate.
